# EUROPEAN PATENT APPLICATION

(11) **EP 2 532 664 A1**
(43) Date of publication of application: **12.12.2012**
(21) Application number: 11075135.1
(22) Date of filing: 09.06.2011
(51) Int. Cl.: C07D 471/14, A61K 31/4375, A61P 35/00

(54) **Substituted indolo [2,3-a] quinolizines in the treatment of cancer**

(71) Applicant: Max-Planck-Gesellschaft zur Förderung der Wissenschaften e.V., 80539 München (DE)
(72) Inventor: Waldmann, Herbert, 44265 Dortmund (DE); Kumar, Kamal, 44137 Dortmund (DE); Hübel, Katja, 44225 Dortmund (DE); Effenberger, Verena, 45147 Essen (DE); Dückert, Heiko, 44149 Dortmund (DE); Menninger, Sascha, 44803 Bochum (DE); Ziegler, Slava, 44269 Dortmund (DE); Bruss, Hanna, 44137 Dortmund (DE); Khedkar, Vivek, Pune, Maharashtra 412210 (IN)
(74) Representative: Arth, Hans-Lothar

(57) **Abstract**

The present invention relates to novel substituted indolo [2,3-*a*] quinolizines of the general formula (I) 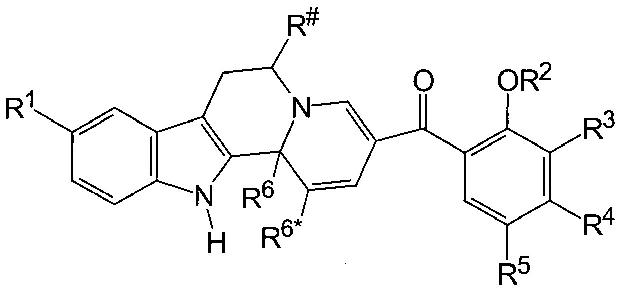 and stereoisomeric forms thereof and/or pharmaceutically acceptable salts of these compounds as well as pharmaceutical compositions containing at least one of these substituted indolo [2,3-*a*] quinolizines together with pharmaceutically acceptable carrier, excipient and/or diluents. Said novel substituted indolo [2,3-*a*] quinolizines have been identified as useful for the prophylaxis and treatment of cancer by the induction of strong mitotic delays, chromosomal misalignments and mitotic tri- and multipolarization leading to cell cycle stop and apoptosis. Furthermore a synthesis for preparation of the substituted indolo [2,3-*a*] quinolizines is disclosed in the present invention.

## Description

The present invention relates to novel substituted indolo [2,3-a] quinolizines and stereoisomeric forms, prodrugs, solvates, hydrates and/or pharmaceutically acceptable salts of these compounds as well as pharmaceutical compositions containing at least one of these substituted indolo [2,3-a] quinolizines together with pharmaceutically acceptable carrier, excipient and/or diluents. Said novel substituted indolo [2,3-a] quinolizines have been identified as useful for the prophylaxis and treatment of cancer by the induction of strong mitotic delays, chromosomal misalignments and mitotic tri-and multipolarization leading to cell cycle stop and apoptosis.
Furthermore a synthesis for preparation of the substituted indolo [2,3-a] quinolizines is disclosed in the present invention.

### Background of the invention

In the treatment of cancer there is an ongoing need for the development of novel substances which are effective to induce cell cycle stop or apoptosis of cancer cells. Disturbances in cell cycle control and apoptosis induction are fundamental characteristics of cancer cells.

Cell division is a fundamental process required throughout the life of all eukaryotes. The biochemical and morphological stages that a cell passes through when stimulated to divide are referred to as the cell cycle, which is conveniently divided into several phases: G1 (Gap-1), S (DNA synthesis), G2 (Gap-2), and M (mitosis). Not all of the cells in the body are actively dividing, i.e., are actively engaged in the cell cycle. Cells may exit the cell cycle at G1 and enter a nonproliferative phase called G0 or quiescence. In response to specific mitogenic stimuli, quiescent cells will exit G0 and reenter the cell cycle at the level of early G 1. The entry of the cells into each stage of the cell cycle in healthy cells is controlled by many regulatory proteins. In malignant cells this control is lost and the cells divide unlimited and independently of growth factor signals.
External agents which interfere with cancer cell cycle progression and apoptosis have a huge potential in the treatment of cancer.
If for example the remaining cell cycle control checkpoints or proteins of the mitotic machinery of a cancer cell are heavily disturbed this can lead to the formation of multipolar spindles, misalignment of chromosomes and in the following to the induction of cell cycle stop and apoptosis. If the disruption of the cell cycle is severe, the cell cycle stop can become permanent or apoptosis can be induced. The result is that tumor progression is stopped as tumor cell division is inhibited or the tumor cell is killed.

Thus, it is the objective of the present invention to provide compounds and pharmaceutical compositions which can be used for the treatment or prophylaxis of cancer, tumors and proliferative diseases.

The objective of the present invention is solved by the teaching of the independent claims. Further advantageous features, aspects and details of the invention are evident from the dependent claims, the description, and the examples of the present application.

In the present invention it was surprisingly found that the indolo[2,3-a]quinolizines of the general formula (I) induce strong mitotic delays, chromosomal misalignments, mitotic tri-and multipolarization, cell cycle stop and apoptosis in cancer cells. Theses inventive compounds thereby are useful for the treatment of cancer, tumors and proliferative diseases.

Also disclosed herein are the structures of the inventive compounds as well as novel methods for their asymmetric synthesis.
In general, the reported synthesis of indoloquinolizine and related alkaloid ring systems in literature are multistep and often tedious processes (R-Corrfa Jr A, Ambrosi. S. Jakupovic, K. Saxena, H. Schwalb. M. Kaiser. H. Waldmann.; Chem. Asian J. 2007, 2, 1109 -1126). Most of these syntheses involve many undesired protection-deprotection steps especially in the asymmetric synthesis of indoloquinolizines. To get an easy and efficient synthesis of the specific indolo[2,3-*a*]quinolizines employing commercially available substrates and without entertaining any protection group chemistry, we resorted to one pot and cascade reaction based synthetic strategy. In the present invention, a one pot cascade synthesis of novel substituted indolo[2,3-*a*]quinolizines and their antitumor activities is described.

### Description of the invention

Accordingly the present invention relates to the substituted indolo[2,3-*a*]quinolizines of the general formula (I): 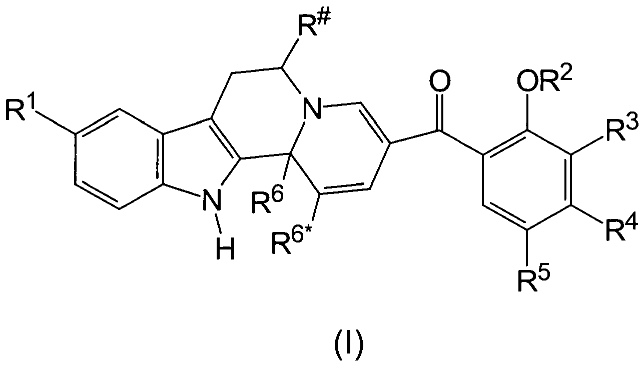 wherein
**R¹** represents -H, -NO₂, -F, -Cl, -Br, -I, -CH₃, -C₂H₅, -C₃H₇, -CH(CH₃)₂, -C₄H₉, -R⁷, -OCH₃, -OC₂H₅, -OC₃H₇, -OCH(CH₃)₂, -OC₄H₉, -OR⁷;
**R², R⁷, R⁸, R⁹ - R¹⁵** represent independently of each other -H, -CH₃, -C₂H₅, -CH₂-CH₂F, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₃H₇, -CH(CH₃)₂, -C₄H₉, -C₅H₁₁, -C₆H₁₃, -C(CH₃)₃, -CH₂-CH(CH₃)₂, -CH(CH₃)-C₂H₅, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₇H₁₅, -C₈H₁₇, -C₃H₆-CH(CH₃)₂, -C₂H₄-CH(CH₃)-C₂H₅, -CH(CH₃)-C_{4H9}, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -C₄H₈-CH=CH₂, -C≡CH, -CC≡CH₃, -CH₂-C≡CH, -C₂H₄-C≡CH;
**R³, R⁴, R⁵, R⁹, R¹⁰, R¹¹, R¹²** represent independently of each other -OR⁸, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)_{2,} -NH-C(=NH)-N(C₃H₇)₂, -NH-C(=NH)-N(cyclo-C₃H₅)₂, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -C₅H₁₁, -C₆H₁₃, -C(CH₃)₃, -CH₂-CH(CH₃)₂, -CH(CH₃)-C₂H₅, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -C₄H₈-CH=CH₂, -C≡CH, -C≡C-CH₃, -CH₂-C≡CH, -C₂H₄-C≡CH, -P(O)(OH)₂, -P(O)(OCH₃)₂, -P(O)(OC₂H₅)₂, -P(O)(OCH(CH₃)₂)₂, -C(OH)[P(O)(OH)₂]₂;
**R³** and **R⁴** may form together a carbocyclic ring represented by the residue 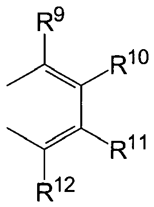 wherein R⁹ to R¹² have the meaning as defined above; and
**R**⁶ and **R**^{6*} represent independently of each other -H, -COOR¹³, -COOR¹⁴, -R¹³, -R¹⁴, -CN;
**R**# represents -H or -COOR¹⁵;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof. Especially the (+) enantiomers are preferred. Below it is disclosed in more detail that chiral compounds of formula (I) can be obtained by the use of chiral acids so that the present invention discloses racemates of the inventive compounds of general formula (I) as well as enantiomerically or diastereomerically pure compounds of general formula (I). The term "(+)-1-01" as used herein refers to the (+)-enantiomer of compound 1-01 while the term "(-)-1-01" as used herein refers to the (-)-enantiomer of compound 1-01. The same nomenclature is used for all other compounds disclosed herein.

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

Preferred are compounds of general formula (I) wherein
**R¹** represents -H, -NO₂, -Cl, -Br, -OH, -R⁷, -OR⁷;
**R³, R⁴** and **R⁵** are hydrogen;
**R⁶** and **R^{6*}** represent independently of each other -H, -COOR¹³, -COOR¹⁴, R¹³, -R¹⁴, -CN;
**R**^{#} represents -H;
**R²**, **R⁷**, **R⁸**, **R¹³** and **R¹⁴** have the meanings as disclosed in claim 1.

Also preferred are compounds of general formula (I) wherein
**R¹** represents -H, -Cl, -Br, -OH, -CH₃, -C₂H₅, -OCH₃, -OC₂H₅;
**R³**, **R⁴ and R⁵** are hydrogen;
**R⁶** and **R^{6*}** represent independently of each other -H, -COOCH₃, -COOC₂H₅, -CH₃, -C₂H₅ or -CN;
**R^{#}** represents -H;
**R²** represents -H, -CH₃ or -C₂H₅.

Moreover preferred are compounds wherein R^{#} represents -H.

Furthermore preferred are compounds wherein R³, R⁴ and R⁵ represent -H as represented by the following general formula (II):

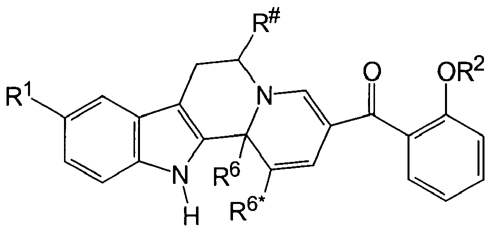

Preferred are compounds according to formula (I), wherein R⁷, R⁸, R¹³ represent independently of each other -H, -CH₃, -C₂H₅, -CH₂-CH₂F, -CH₂-CF₃, -CH₂-CH₂Cl, -CH₂-CH₂Br, -CH₂-CH₂l.

Also preferred are compounds wherein R¹ represent -OR⁷. Still preferred are compounds wherein R⁷, R⁸, R¹³ represent independently of each other -CH₃ or -C₂H_{5.}

Also preferred are compounds wherein both groups R⁶ represent -COOCH₃ or -COOC₂H₅.

Most preferred are the following compounds according to the general formula (I):

| Compound | R¹ | R² | R³ | R⁴ | R⁵ | R⁶ |
|---|---|---|---|---|---|---|
| number | | | | | | |
| 1-01 | H | H | H | H | H | CO₂CH₃ |
| 1-02 | H | H | H | H | H | CO₂C₂H₅ |
| 1-03 | H | H | H | H | CH₃ | CO₂C₂H₅ |
| 1-04 | OCH₃ | H | H | H | H | CO₂CH₃ |
| 1-05 | OCH₃ | H | H | H | Br | CO₂CH₃ |
| 1-06 | H | H | H | H | Cl | CO₂CH₃ |
| 1-07 | H | H | H | H | Br | CO₂CH₃ |
| 1-08 | H | H | H | H | CH₃ | CO₂CH₃ |
| 1-09 | H | H | H | H | iPr | CO₂CH₃ |
| 1-10 | H | H | Cl | H | Cl | CO₂CH₃ |
| 1-11 | H | H | Br | H | Br | CO₂CH₃ |
| 1-12 | H | H | H | CH₃ | Cl | CO₂CH₃ |
| 1-13 | OCH₃ | H | H | H | H | CO₂C₂H₅ |
| 1-14 | H | H | H | H | Br | CO₂C₂H₅ |
| 1-15 | H | H | Cl | H | Cl | CO₂C₂H₅ |
| 1-16 | OCH₃ | H | H | H | CH₃ | CO₂C₂H₅ |
| 1-17 | OCH₃ | H | H | H | iPr | CO₂C₂H₅ |
| 1-18 | H | H | H | CH₃ | Cl | CO₂C₂H₅ |
| 1-19 | H | H | H | H | Cl | CO₂C₂H₅ |
| 1-20 | OCH₃ | H | H | H | Cl | CO₂C₂H₅ |
| 1-21 | H | H | Ph | | H | CO₂CH₃ |
| 1-22 | OCH₃ | H | H | H | Br | CO₂C₂H₅ |
| 1-23 | H | H | CH₃ | OBn | H | CO₂CH₃ |
| 1-24 | H | CH₃ | H | H | H | CO₂CH₃ |

The chemical IUPAC names of the most preferred compounds read as follows:
Compound 1-01: 3-(2-Hydroxybenzoyl)-7,12-dihydro-*6H*-indolo[2,3-*a*]quinolizine-1,12b-dicarboxylic acid dimethyl ester,
Compound 1-02: 3-(2-Hydroxybenzoyl)-7,12-dihydro-*6H*-indolo[2,3-*a*]quinolizine-1,12b-dicarboxylic acid diethyl ester,
Compound 1-03: 3-(2-Hydroxybenzoyl)-7,12-dihydro-*6H*-indolo[2,3-*a*]quinolizine-1,12b-dicarboxylic acid diethyl ester,
Compound 1-04: 3-(2-Hydroxy-5-methylbenzoyl)-7,12-dihydro-*6H*-indolo[2,3-*a*]quinolizine-1,12b-dicarboxylic acid diethyl ester,
Compound 1-05: 3-(5-Bromo-2-hydroxybenzoyl)-9-methoxy-7,12-dihydro-*6H-*indolo[2,3-*a*]quinolizine-1,12b-dicarboxylic acid dimethyl ester,
Compound 1-06: 3-(5-Chloro-2-hydroxybenzoyl)-7,12-dihydro-*6H*-indolo[2,3-*a*]quinolizine-1,12b-dicarboxylic acid dimethyl ester,
Compound 1-07: 3-(5-Bromo-2-hydroxybenzoyl)-7,12-dihydro-*6H*-indolo[2,3-*a*]quinolizine-1,12b-dicarboxylic acid dimethyl ester,
Compound 1-08: 3-(2-Hydroxy-5-methylbenzoyl)-7,12-dihydro-*6H*-indolo[2,3-*a*]quinolizine-1,12b-dicarboxylic acid dimethyl ester,
Compound 1-09: 3-(2-Hydroxy-5-iso-propylbenzoyl)-7,12-dihydro-*6H*-indolo[2,3-*a*]quinolizine-1,12b-dicarboxylic acid dimethyl ester,
Compound 1-10: 3-(3,5-Dichloro-2-hydroxybenzoyl)-7,12-dihydro-*6H*-indolo[2,3-*a*]quinolizine-1,12b-dicarboxylic acid dimethyl ester,
Compound 1-11: 3-(3,5-Dibromo-2-hydroxybenzoyl)-7,12-dihydro-*6H*-indolo[2,3-*a*]quinolizine-1,12b-dicarboxylic acid dimethyl ester,
Compound 1-12: 3-(5-Chloro-2-hydroxy-4-methylbenzoyl)-7,12-dihydro-*6H*-indolo[2,3-*a*]quinolizine-1,12b-dicarboxylic acid dimethyl ester,
Compound 1-13: 3-(2-Hydroxybenzoyl)-9-methoxy-7,12-dihydro-*6H*-indolo[2,3-*a*]quinolizine-1,12b-dicarboxylic acid diethyl ester,
Compound 1-14: 3-(5-Bromo-2-hydroxybenzoyl)-7,12-dihydro-*6H*-indolo[2,3-*a*]quinolizine-1,12b-dicarboxylic acid diethyl ester,
Compound 1-15: 3-(3,5-Dichloro-2-hydroxybenzoyl)-7,12-dihydro-*6H*-indolo[2,3-*a*]quinolizine-1,12b-dicarboxylic acid diethyl ester,
Compound 1-16: 3-(2-Hydroxy-5-methylbenzoyl)-9-methoxy-7,12-dihydro-*6H-*indolo[2,3-*a*]quinolizine-1,12b-dicarboxylic acid diethyl ester,
Compound 1-17: 3-(2-Hydroxy-5-iso-propylbenzoyl)-9-methoxy-7,12-dihydro-*6H-*indolo[2,3-*a*]quinolizine-1,12b-dicarboxylic acid diethyl ester,
Compound 1-18: 3-(5-Chloro-2-hydroxy-4-methylbenzoyl)-7,12-dihydro-*6H*-indolo[2,3-*a*]quinolizine-1,12b-dicarboxylic acid diethyl ester,
Compound 1-19: 3-(5-Chloro-2-hydroxybenzoyl)-7,12-dihydro-*6H*-indolo[2,3-*a*]quinolizine-1,12b-dicarboxylic acid diethyl ester,
Compound 1-20: 3-(5-Chloro-2-hydroxybenzoyl)-9-methoxy-7,12-dihydro-*6H-*indolo[2,3-*a*]quinolizine-1,12b-dicarboxylic acid diethyl ester,
Compound 1-21: 3-(1-Hydroxynaphthalene-2-carbonyl)-7,12-dihydro-*6H*-indolo[2,3-*a*]quinolizine-1,12b-dicarboxylic acid dimethyl ester,
Compound 1-22: 3-(5-Bromo-2-hydroxybenzoyl)-9-methoxy-7,12-dihydro-*6H-*indolo[2,3-*a*]quinolizine-1,12b-dicarboxylic acid diethyl ester,
Compound 1-23: 3-(4-Benzyloxy-2-hydroxy-5-methylbenzoyl)-7,12-dihydro-*6H-*indolo[2,3-*a*]quinolizine-1,12b-dicarboxylic acid dimethyl ester,
Compound 1-24: 3-(2-Methoxybenzoyl)-7,12-dihydro-*6H*-indolo[2,3-*a*]quinolizine-1,12b-dicarboxylic acid dimethyl ester.
Compound 1-25: Dimethyl-3-(2-hydroxybenzoyl)-9-hydroxy-6,7,12,12b-tetrahydroindolo[2,3-*a*]quinolizine-1,12b-dicarboxylate
Compound 1-26: Dimethyl-3-(2-hydroxybenzoyl)-9-methyl-6,7,12,12b-tetrahydro-indolo[2,3-*a*]quinolizine-1,12b-dicarboxylate
Compound 1-27: Dimethyl-9-bromo-3-(2-hydroxybenzoyl)-6,7,12,12b-tetrahydro-indolo[2,3-*a*]quinolizine-1,12b-dicarboxylate
Compound 1-28: (6*S*)-trimethyl 3-(2-hydroxybenzoyl)-6,7,12,12b-tetrahydroindolo[2,3-a]quinolizine-1,6,12b-tricarboxylate
Compound 1-29: 3-(2-Hydroxybenzoyl)-7,12-dihydro-*6H*-indolo[2,3-*a*]quinolizine-1-carboxylic acid methyl ester,
Compound 1-30: 3-(5-Chloro-2-hydroxybenzoyl)-7,12-dihydro-*6H*-indolo[2,3-*a*]quinolizine-1-carboxylic acid methyl ester
Compound 1-31: 3-(2-Hydroxybenzoyl)-7,12-dihydro-*6H*-indolo[2,3-*a*]quinolizine-1-carbonitrile
Compound 1-32: 3-(5-Chloro-2-hydroxybenzoyl)-7,12-dihydro-*6H*-indolo[2,3-*a*]quinolizine-1-carbonitrile
Compound 1-33: 3-(2-Hydroxybenzoyl)-12b-methyl-7,12-dihydro-*6H*-indolo[2,3-*a*]quinolizine-1-carboxylic acid methyl ester
Compound 1-34: 3-(5-Chloro-2-hydroxybenzoyl)-12b-methyl-7,12-dihydro-*6H-*indolo[2,3-*a*]quinolizine-1-carboxylic acid methyl ester
Compound 1-35: 3-(2-Hydroxybenzoyl)-12b-methyl-7,12-dihydro-*6H*-indolo[2,3-*a*]quinolizine-1-carbonitrile
Compound 1-36: 3-(5-Chloro-2-hydroxybenzoyl)-12b-methyl-7,12-dihydro-*6H-*indolo[2,3-*a*]quinolizine-1-carbonitrile
Compound 1-37: 12b-ethyl 1-methyl 3-(2-hydroxybenzoyl)-6,7,12,12b-tetrahydroindolo[2,3-a]quinolizine-1,12b-dicarboxylate
Compound 1-38: 12b-ethyl 1-methyl 3-(5-chloro-2-hydroxybenzoyl)-6,7,12,12b-tetrahydroindolo[2,3-a]quinolizine-1,12b-dicarboxylate
Compound 1-39: ethyl 1-cyano-3-(2-hydroxybenzoyl)-6,7,12,12b-tetrahydroindolo[2,3-a]quinolizine-12b-carboxylate
Compound 1-40: ethyl 3-(5-chloro-2-hydroxybenzoyl)-1-cyano-6,7,12,12b-tetrahydroindolo[2,3-a]quinolizine-12b-carboxylate
Compound 1-41: 3-(2-Hydroxybenzoyl)-9-methoxy-7,12-dihydro-*6H*-indolo[2,3-*a*]quinolizine-1-carboxylic acid methyl ester
Compound 1-42: methyl 3-(2-hydroxybenzoyl)-12b-methyl-6,7,12,12b-tetrahydroindolo[2,3-a]quinolizine-1-carboxylate
Compound 1-43: 3-(2-Hydroxybenzoyl)-9-methoxy-7,12-dihydro-*6H*-indolo[2,3-*a*]quinolizine-1-carbonitrile
Compound 1-44: 3-(5-Chloro-2-hydroxybenzoyl)-9-methoxy-7,12-dihydro-*6H-*indolo[2,3-*a*]quinolizine-1-carbonitrile
Compound 1-45: 3-(2-Hydroxybenzoyl)-9-methoxy-12b-methyl-7,12-dihydro-*6H-*indolo[2,3-*a*]quinolizine-1-carboxylic acid methyl ester
Compound 1-46: 3-(5-Chloro-2-hydroxybenzoyl)-9-methoxy-12b-methyl-7,12-dihydro-*6H*-indolo[2,3-*a*]quinolizine-1-carboxylic acid methyl ester
Compound 1-47: 3-(2-Hydroxybenzoyl)-9-methoxy-12b-methyl-7,12-dihydro-*6H-*indolo[2,3-*a*]quinolizine-1-carbonitrile
Compound 1-48: 3-(5-Chloro-2-hydroxybenzoyl)-9-methoxy-12b-methyl-7,12-dihydro-*6H*-indolo[2,3-*a*]quinolizine-1-carbonitrile
Compound 1-49: 3-(2-Hydroxybenzoyl)-7,12-dihydro-*6H*-indolo[2,3-*a*]quinolizine, Compound 1-50: 3-(2-Hydroxybenzoyl)-12b-methyl-7,12-dihydro-*6H*-indolo[2,3-*a*]quinolizine
Compound 1-51: 3-(2-Hydroxybenzoyl)-7,12-dihydro-*6H*-indolo[2,3-*a*]quinolizine-12b-carboxylic acid ethyl ester,
Compound 1-52: 3-(2-Hydroxybenzoyl)-9-methoxy-7,12-dihydro-*6H*-indolo[2,3-*a*]quinolizine
Compound 1-53: 3-(2-Hydroxybenzoyl)-9-methoxy-12b-methyl-7,12-dihydro-*6H-*indolo[2,3-*a*]quinolizine

The present invention also comprises pharmaceutically acceptable salts of the compounds according to the general formula (I), all stereoisomeric forms of the compounds according to the general formula (I) as well as solvates, especially hydrates or prodrugs thereof.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

The compounds of the general formula (I) exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s).

Another aspect of the present invention relates to the use of the inventive substituted indolo[2,3-a]quinolizines as drugs, i.e. as pharmaceutically active agents applicable in medicine.

Surprisingly it was found that the above-mentioned indolo[2,3-a]quinolizines as well as the pharmaceutical compositions comprising said indolo[2,3-a]quinolizines are useful for treatment or prophylaxis of cancer, tumors and proliferative diseases as they induce cell cycle stop and apoptosis.

Thus, the indolo[2,3-a]quinolizines compounds of the present invention can be used for prophylaxis and treatment of cancer, tumors and proliferative diseases or for the preparation of a pharmaceutical formulation for prophylaxis and treatment of cancer, tumors and proliferative diseases.
Examples for cancer types which can be treated and/or prevented by the inventive compounds are selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Apoptosis or programmed cell death is defined as a mechanism which occurs after sufficient cellular damage. It is characteristically different from cell necrosis in morphology and biochemistry.

Apoptosis results in the condensation of the nuclues, and the cell shrinks. Visible in apoptotic cells is chromosomal fragmentation due to the controlled digestion of DNA by apoptosis DNAses. Cytoplasmic blebbing and apoptotic bodies are also seen during apoptosis.

The end result of apoptosis is cell death without inflammation of the surrounding tissue. Research on apoptosis has increased substantially since the early 1990s. In addition to its importance as a biological phenomenon, defective apoptotic processes have been implicated in an extensive variety of diseases. Excessive apoptosis causes hypotrophy, such as in ischemic damage, whereas an insufficient amount results in uncontrolled cell proliferation, such as cancer.

Apoptosis is an integral part of tissue development. Development of an organ or tissue is often preceded by the extensive division and differentiation of a particular cell, the resultant mass is then "pruned" into the correct form by apoptosis. Unlike cellular death caused by injury, apoptosis results in cell shrinkage and fragmentation. This allows the cells to be efficiently phagocytosed and their components reused without releasing potentially harmful intracellular substances into the surrounding tissue.

The process of apoptosis is controlled by a diverse range of cell signals, which may originate either extracellularly or intracellularly. Extracellular signals may include hormones, growth factors, nitric oxide or cytokines and therefore must either cross the plasma membrane or transduce to affect a response. These signals may positively or negatively induce apoptosis; in this context the binding and subsequent initiation of apoptosis by a molecule is termed positive, whereas the active repression of apoptosis by a molecule is termed negative.

Intracellular apoptotic signalling is a response initiated by a cell in response to stress, and may ultimately result in cell suicide. The binding of nuclear receptors by glucocorticoids, heat, radiation, nutrient deprivation, viral infection, and hypoxia are all factors that can lead to the release of intracellular apoptotic signals by a damaged cell.

Before the actual process of cell death is carried out by enzymes, apoptotic signals must be connected to the actual death pathway by way of regulatory proteins. This step allows apoptotic signals to either culminate in cell death, or be aborted should the cell no longer need to die. Several proteins are involved, however two main methods of achieving regulation have been identified; targeting mitochondria functionality, or directly transducing the signal via adapter proteins to the apoptotic mechanisms. The whole preparation process requires energy and functioning cell machinery.

The mitochondria are essential to multicellular life. Without them, a cell ceases to respire aerobically and quickly dies - a fact exploited by some apoptotic pathways. Apoptotic proteins that target mitochondria affect them in different ways; they may cause mitochondrial swelling through the formation of membrane pores, or they may increase the permeability of the mitochondrial membrane and cause apoptotic effectors to leak out. Nitric oxide (NO) is able to induce apoptosis by helping to dissipate the membrane potential of mitochondria and therefore make it more permeable.

Mitochondrial proteins known as SMACs (second mitochondria-derived activator of caspases) are released into the cytosol following an increase in permeability. SMAC binds to inhibitor of apoptosis proteins (IAPs) and deactivates them, preventing the IAPs from arresting the apoptotic process and therefore allowing apoptosis to proceed. IAP also normally suppresses the activity of a group of cysteine proteases called caspases, which carry out the degradation of the cell, therefore the actual degradation enzymes can be seen to be indirectly regulated by mitochondrial permeability.

Although many pathways and signals lead to apoptosis, there is only one mechanism that causes the death of the cell in this process; after the appropriate stimulus has been received by the cell and the necessary controls exerted, a cell will undergo the organized degradation of cellular organelles by activated proteolytic caspases. A cell undergoing apoptosis shows a characteristic morphology that can be observed with a microscope:Cell shrinkage and rounding due to the breakdown of the proteinaceous cytoskeleton by caspases. The cytoplasm appears dense, and the organelles appear tightly packed. Chromatin undergoes condensation into compact patches against the nuclear envelope in a process known as pyknosis, a hallmark of apoptosis.The nuclear envelope becomes discontinuous and the DNA inside it is fragmented in a process referred to as karyorrhexis. The nucleus breaks into several discrete chromatin bodies or nucleosomal units due to the degradation of DNA. The cell membrane shows irregular buds. The cell breaks apart into several vesicles called apoptotic bodies, which are then phagocytosed. Apoptosis can occur when a cell is damaged beyond repair, infected with a virus, or undergoing stress conditions such as starvation. DNA damage from ionizing radiation or toxic chemicals can also induce apoptosis via the actions of the tumor-suppressing gene p53. The "decision" for apoptosis can come from the cell itself, from the surrounding tissue, or from a cell that is part of the immune system. In these cases apoptosis functions to remove the damaged cell, preventing it from sapping further nutrients from the organism, or to prevent the spread of viral infection.

The induction of apoptosis as disclosed for the compounds of the present invention is useful in the treatment of cancer by inducing the death of the malignant cells. Since all kinds of cancer cells are destroyable through the induction of apoptosis and cell cycle stop, all different kinds of cancer and abnormal proliferating cells can be treated with the compounds of the present invention.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions further comprise at least one active indolo[2,3-a]quinolizine of the general formula (I).

### Chemical synthesis novel indolo[2,3-a]quinolizines:

The cascade synthesis described here (GP I) is one of the longest ever known sequences of different reactions happening in one pot from all commercially available substrates, thereby providing a practical, efficient and easy synthesis of indoloquinolizines of general formula (I).

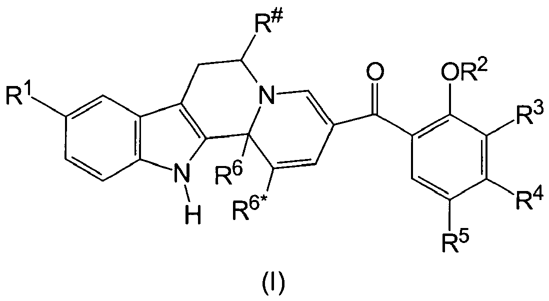

### Thereby, 3-formylchromone (compound A)

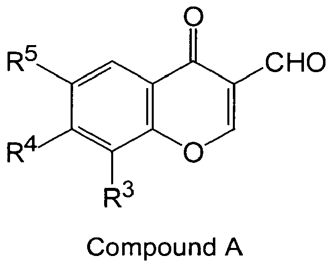

dissolved in an organic aprotic solvent such as toluene, is reacted with a in situ generated complex of triphenylphosphine and acetylenedicarboxylate (compound B) 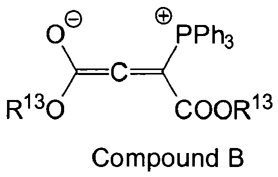 and compound C is formed

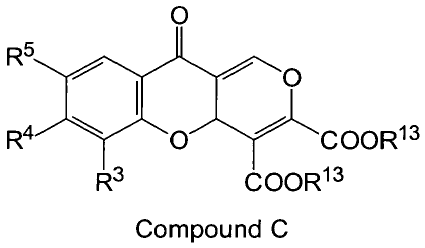

Compound C is further reacted to the novel compounds of general formula (I) by the addition of tryptamine (compound D) 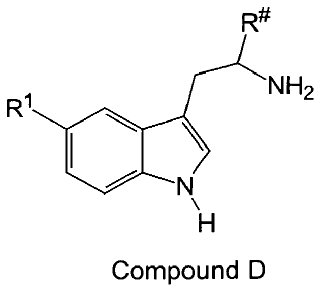 for 5-10 minutes followed by the addition of an acid and preferably an organic chiral acid such as camphor sulphonic acid (CSA) or chiral phosphoric acids (e.g. compound E) and further stirring the reaction mixture for 5-30 minutes leading to the formation of indoloquinolizines of general formula (I), wherein R² is hydrogen in good yields.

Optionally the free hydroxy group (-OR² with R² = H) can further be alkylated in order to obtain the final compounds of general formula (I) wherein R² is different from hydrogen.

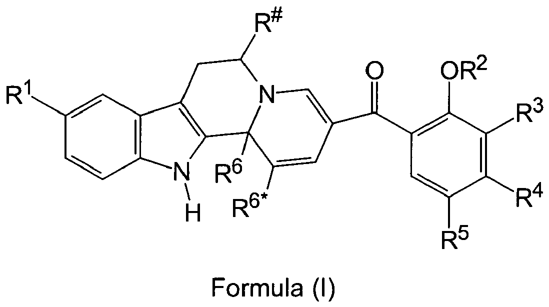

In a preferred embodiment chiral compounds of formula (I) are synthesized by the use of chiral acids for the reaction of compound C and compound D and the subsequent rearrangement to the final products of general formula (I) wherein R² is hydrogen. Examples of chiral acids are chiral organic phosphoric acids such as Compound E mentioned below, tartaric acid, malic acid or other chiral organic acids which are used as pure enantiomer or pure diastereomer in order to produce enantiomerically and/or diastereomerically pure products. By this method which is described in more detail below, an enantiomeric excess (ee) as well as in case of a substituent with another chiral center a diastereomeric excess (de) of at least 80%, more preferably of at least 85%, still more preferably of at least 90% and most preferably of at least 95% can be obtained.

The enantioselective and/or diastereoselective synthesis is important for these inventive compounds, where the single enantiomers or single disatereomers display enantiomer specific or diastereomer specific biological activities, for instance, strong mitotic delays, chromosomal misalignments and mitotic tri- and multipolarization at micro molar concentrations.

However it can be stated that the inventive compounds show in general therapeutically valuable biological activities, for instance, strong mitotic delays, chromosomal misalignments and mitotic tri- and multipolarization at micro molar concentrations. Moreover their ability to induce apoptosis and reducing cell proliferation in different cell lines make them to good anticancer drugs.

Another way to prepare the compounds of general formula (IA) (GP IA = Formula I with R^{#} = H) is as follows.

Reacting a compound F of the following general formula 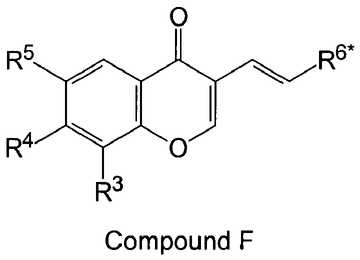 with the amine compound G of the following general formula 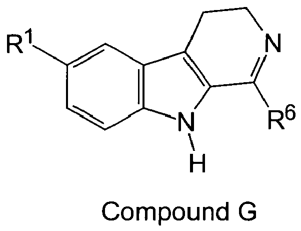 in order to obtain the compounds of general formula (IA), wherein R² is hydrogen in good yields.

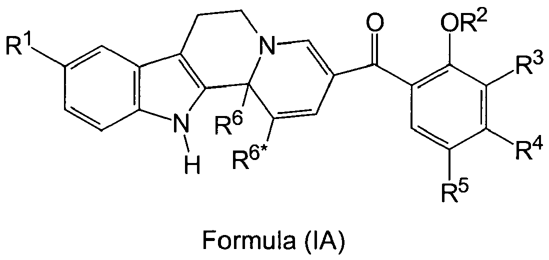

Optionally the free hydroxy group (-OR² with R² = H) can further be alkylated in a second step in order to obtain the final compounds of general formula (IA) wherein R² is different from hydrogen.

### Description of the figures:

- **Figure 1:**: FACS analysis. Cell cycle analysis of HeLa cells treated with DMSO (a), 1 µM nocodazole (b), 25µM (-)-1-01 (c), 50 µM (-)-1-01 (d).
- **Figure 2:**: Effect of compound (-)-1-01 on different cell lines. HeLa, MCF7, SW480 and HT29 cells were seeded on cover glasses and treated with 25 µM (-)-1-01 for 18h. The cells were then fixed and stained for DNA and α-tubulin
- **Figure 3:**: Effect of compound (-)-1-01 and compound (-)-1-04 on the cell proliferation. Cells were treated with increasing compound concentrations for 19 hours. Cell proliferation was measured using the WST-1 reagent. Results are expressed as percent of control (DMSO treated cells). Data are representative for three independent experiments.
- **Figure 4:**: Effect of compound (-)-1-01 and compound (-)-1-04 on the apoptosis in HeLa (Fig. 4A), HCT116 (Fig. 4B), PC3 (Fig. 4C), Saos2 (Fig. 4D) and HT29 (Fig. 4E) cells. Cells were incubated for 42 hours with different compound concentrations and the caspase-3/7 activity was determined by means of Apo-ONE homogeneous caspase-3/7 assay. Results are expressed as percent of control (DMSO treated cells). STS refers to staurosporine. Data are representative for three independent experiments.

### General Examples

### General procedure I (GP I) for the cascade synthesis of indolo[2,3-a]quinolizine

1 mmol of a 3-formylchromone derivative was dissolved in toluene (10 ml) by heating and 1.3 eq. of acetylenedicarboxylate was added followed by 0.6 eq of triphenylphosphine. Monitoring by TLC (DCM/MeOH 100:1) showed completion typically after 5-10 minutes. Then 1.1 eq of the tryptamine derivative was added. After the tryptamine has dissolved 1.5 eq. camphorsulfonic acid was added. Monitoring by TLC (cyclohexane - ethyl acetate 60:40) showed the reaction to be complete after 5-30 minutes and the reaction mixture was directly subjected to column chromatography (30 g silica gel, cyclohexane - ethyl acetate 80:20). Removal of the solvent in vacuo evaporator gave the product as yellow solid. In case the purity was not sufficient the product was recrystallized from methanol.

### General procedure IA (GP IA) for the cascade synthesis of indolo[2,3-a]quinolizine

The compound G / imine (1.2 eq., 0.6 mmol) was dissolved in toluene (5mL) in a Radley Reaction Glass Tube, and the solution was heated to 80°C. After full dissolution of the imine, compound F, the chromone-diene (1.0 eq., 0.5 mmol) and Zn catalyst (20 mol%, 0.1 mmol) were added to the mixture, and was left stirring overnight. When TLC or LCMS showed total disparition of the chromone-diene, the solution was allowed to cool down to room temperature. 25 mL of DCM were added to the solution and washed twice with brine, and the organic layer was dried over Na₂SO₄ and reduced by vacuum. The residue was then purified through preparative HPLC (10 to 100% water in acetonitrile on C18 nucleodur column) with a retention time of 6min

### Characterization of compounds:

General: Chemicals used were purchased from Sigma-Aldrich, Acros or Alfa Aesar. Reactions were carried out in dry solvents under argon. NMR spectra were recorded on a Bruker Avance 400, Bruker Avance 500, or Varian Mercury-Vx 400. The spectra were calibrated on the solvent signals (Chloroform-d₁: δ=7.24 ppm for ¹H, δ=77.23 ppm for ¹³C ; Dimethylsulfoxide-d₆: δ=2.50 ppm for ¹H, d=39.51 ppm for ¹³C; Methanol-*d*₄: δ=3.31 ppm for ¹H, δ=9.15 ppm for ¹³C). Optical rotations were determined using a Schmidt+Haensch HH8. Melting points were measured on a Büchi B-540. TLCs were run on TLC aluminium sheets Silica gel 60 F254 by Merck. HRMS were recorded using electrospray ionization on an LTQ Orbitrap coupled to an Accela HPLC-System with a Hypersil GOLD column, both instruments and column from Thermo Electron. Separation of enantiomers was in general performed on an Agilent 1100 using a Daicel IC-column and isohexane /methylene chloride / ethanol (60:40:0.8) as mobile phase. Separation of the enantiomers of the molecules 1-1a [(+)-1-1a and (-)-1-1a] and 1-4 [(+)-1-4 and (-)-1-4] was performed as described above on an Agilent 1100 using a Daicel IC-column and isohexane / methylene chloride / ethanol (60:40:0.8) as mobile phase.

### Asymmetric cascade synthesis of dimethyl-3-(2-hydroxybenzoyl)-6,7,12,12b-tetrahydroindolo[2,3a]quinolizine-1,12b-dicarboxylate (1-01)

Although a number of asymmetric reactions for the construction of optically active quinolizidines and related ring systems have been developed, these strategies are in general target specific, multistep syntheses relying on starting material from the chiral pool. On the other hand only few strategies exploring asymetric catalyses have been described.
Asymetric catalyses generating an entantiomeric excess of compounds of the general formula (I) can lead to the production of compounds which are of higher efficiency in the induction of cell cycle stop and apoptosis than their enantiomeric counterparts.

The substituted indolo[2,3-a]quinolizine 1-01 has only one chiral center which is generated during the Pictet-Spengler cyclization. Bronsted acid catalysis using chiral phosphoric acids has been reported to induce enantioselectivity in the Pictet-Spengler reaction. Therefore, employing a shorter version of the long cascade sequence described above and using chiral phosphoric acid Compound E as catalyst, an enantioselective route to the indolo[2,3-a]quinolizine 1-01 was developed. Thus a solution of adduct Compound C where R⁵ = R⁴ = R³ = H, and R¹³ = Me, and tryptamine at 90°C was stirred for ten minutes and after cooling down an orange-yellow solid intermediate was purified quickly by flash column chromatography. The later was dried under vacuum and was dissolved in toluene at 50° C and to this solution was added the chiral phosphoric acid catalyst 21 (10 mol%) and the reaction mixture was stirred at the same temperature for 48 h which yielded 1-01 in 88% yield and 63% ee.

### Procedure for the asymmetric cascade synthesis of dimethyl-3-(2-hydroxybenzoyl)-6,7,12,12b-tetrahydroindolo[2,3a]quinolizine-1,12b-dicarboxylate (1-01)

To a solution of 315 mg (1.00 mmol) annulation product (compound C, wherein R⁶ = Me) 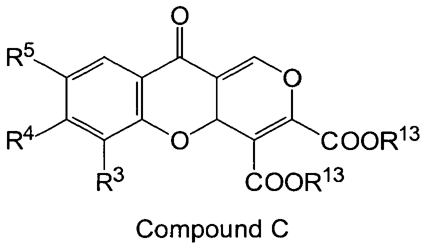 in toluene (10 ml) was added 184 mg (1.15 mmol) tryptamine.

After stirring for 10 minutes at 90°C TLC (Silica gel, CH/EA 60:40) showed complete consumption of the compound C. After cooling to room temperature, the reaction mixture was subjected to column chromatography (Silica gel, CH/EA 70:30). The orange-yellow product was obtained as a mixture of two isomers (with one major compound) and dried under vacuum. 10.0 mg (21.8 µmol) of the intermediate was dissolved in toluene (2 ml) heated to 50°C and 2.2 µmol of the chiral catalyst chiral phosphoric acid (Compound E) was added.

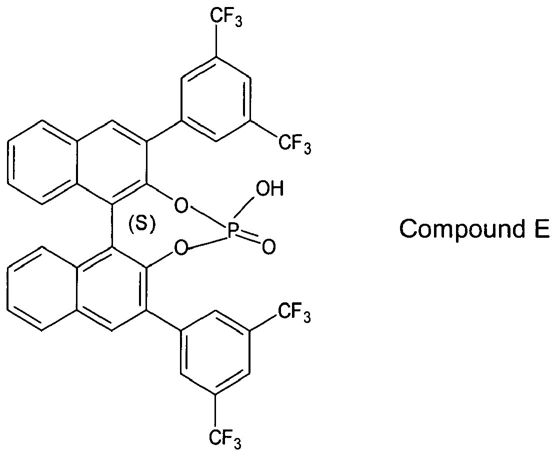

After two days TLC (silica gel, cyclohexane - ethyl acetate 60:40) showed complete conversion to the final product and the reaction mixture was subjected to column chromatography (silica gel, cyclohexane - ethyl acetate 85:15) and the product 1-01 was obtained in 88% yield. Enantiomeric excess was determined by chiral HPLC (IC column, H-hexane/dichloromethane/ethanol 60:40:0.8) with (+)-1-01 as major enantiomer.

### Example 1

Preparation of Dimethyl-3-(2-hydroxybenzoyl)-6,7,12,12b-tetrahydroindolo[2,3a] quinolizine-1,12b-dicarboxylate (1-01 a):

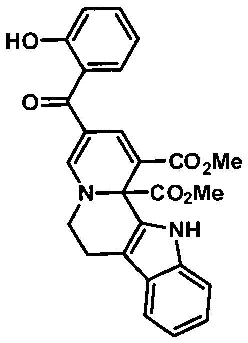

According to the general procedure 1.0 mmol (174 mg) 3-formylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(*R*)-(-)-camphorsulfonic acid in toluene (5 ml). After purification 0.86 mmol (396 mg, 86% yield) of the desired compound was isolated as a yellow solid; Rf = 0.43 (cyclohexane - ethyl acetate 60:40); mp 258°C (decomposition);
¹H NMR (400 MHz, CDCl₃) δ 11.25 (s, 1H), 9.05 (s, 1H), 7.92 (d, *J* = 1.5, 1H), 7.59 (d, *J* = 1.5, 1H), 7.45 (d, *J* = 7.9, 1H), 7.45 (dd, *J* = 7.9, 1.6, 1H), 7.42 - 7.37 (m, 1H), 7.32 (d, *J* = 8.2, 1H), 7.16 (ddd, *J* = 8.2, 7.1, 1.1, 1H), 7.05 (dd, *J* = 11.0, 3.9, 1H), 6.97 (dd, *J* = 8.3, 1.0, 1H), 6.89 - 6.81 (m, 1H), 3.84 (s, 3H), 3.82 (s, 4H), 3.73 (dd, *J* = 13.2, 5.0, 2H), 3.09 (ddd, *J* = 15.6, 12.0, 5.7, 1H), 2.98 (dd, *J* = 15.5, 3.4, 1H);
¹³C NMR (101 MHz, CDCl₃) δ 192.0, 170.0, 168.7, 161.3, 153.5, 136.3, 136.1, 134.5, 131.8, 130.4, 126.1, 123.0, 119.9, 119.9, 118.6, 118.4, 118.3, 113.4, 112.0, 108.2, 106.0, 68.0, 53.9, 52.6, 52.5, 23.2;
HRMS: Calcd for C₂₆H₂₃O₆N₂ [M+H]⁺: 459.15506, Found: 459.15454.

The enantiomers of **1-01** i.e. **(-)-1-01** and **(+)-1-01** were purified by HPLC on an Agilent 1100 using a Daicel IC-column and isohexane / methylene chloride / ethanol (60:40:0.8) as mobile phase. **(-)-1-01:** [α]^{D}₂₀, -17.6° (c = 0.408 g/100ml); **(+)-1-01:** [α]^{D}₂₀, + 13.7° (c = 0.495 g/100ml).

### Example 2

Preparation of Diethyl-3-(2-hydroxybenzoyl)-6,7,12,12b-tetrahydroindolo[2,3-*a*] quinolizine-1,12b-dicarboxylate (1-02):

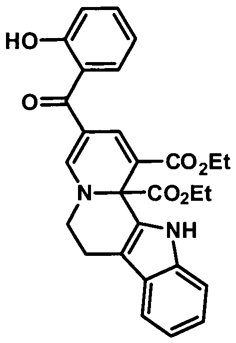

According to the general procedure 1.0 mmol (174 mg) 3-formylchromone was reacted with 1.3 mmol (221 mg) diethylacetylenedicarboxylate, 0.6 mmol (157 mg) triphenylphosphine, 1.1 mmol (176 mg) tryptamine and 1.5 mmol (348 mg) 1-(R)-(-)-camphorsulfonic acid in toluene (10 ml). After purification 0.88 mmol (428 mg, 88% yield) of the desired compound was isolated as a yellow solid; Rf = 0.44 (cyclohexane - ethyl acetate 60:40); mp 209 °C; ¹H NMR (400 MHz, CDCl₃) δ 11.26 (s, 1H), 9.11 (s, 1H), 7.93 (d, *J* = 1.5, 1H), 7.59 (d, *J* = 1.5, 1H), 7.50 - 7.37 (m, *J* = 15.6, 9.9, 7.4, 1.2, 3H), 7.33 (dt, *J* = 8.2, 0.9, 1H), 7.18 (ddd, *J* = 8.2, 7.1, 1.2, 1H), 7.09 (ddd, *J* = 8.0, 7.1, 1.0, 1H), 7.00 (dd, *J* = 8.3, 0.9, 1H), 6.85 (ddd, *J* = 7.8, 7.3, 1.2, 1H), 4.38 - 4.19 (m, 4H), 3.93 - 3.78 (m, 1H), 3.71 (dd, *J* = 13.2, 4.9, 1H), 3.15 *(ddd, J =* 15.5, 12.0, 5.6, 1H), 2.93 (dd, *J* = 15.5, 3.5, 1H), 1.34 (t, *J* = 7.1, 3H), 1.25 (t, *J* = 7.1, 3H); ¹³C NMR (101 MHz, CDCl₃) δ 192.07, 169.28, 168.39, 161.36, 153.62, 136.14, 135.83, 134.56, 131.98, 130.42, 126.11, 122.97, 120.03, 119.87, 118.61, 118.43, 118.33, 114.19, 111.99, 108.19, 106.12, 68.22, 63.24, 61.77, 52.41, 23.33, 14.48, 14.28; HRMS: Calcd for C₂₈H₂₇O₆ [M+H]⁺: 487.18636 Found: 487.18574.

### Example 3

Preparation of Diethyl-3-(2-hydroxy-5-methylbenzoyl)-6,7,12,12b-tetrahydroindolo-[2,3-*a*]quinolizine-1,12b-dicarboxylate (1-03):

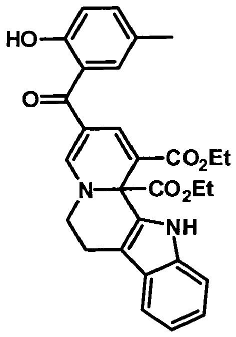

According to the general procedure I, 1.0 mmol (188 mg) 3-formyl-6-methylchromone was reacted with 1.3 mmol (221 mg) diethylacetylenedicarboxylate, 0.6 mmol (157 mg) triphenylphosphine, 1.1 mmol (176 mg) tryptamine and 1.5 mmol (348 mg) 1-(*R*)-(-)-camphorsulfonic acid in toluene (10 ml). After purification 0.65 mmol (327 mg, 65% yield) of the desired compound was isolated as a yellow solid; Rf = 0.45 (cyclohexane - ethyl acetate 60:40); mp 208°C; ¹H NMR (400 MHz, CDCl₃) δ 11.02 (s, 1H), 9.11 (s, 1H), 7.93 (d, *J* = 1.5, 1H), 7.61 (d, *J* = 1.5, 1H), 7.47 (dd, *J* = 7.9, 0.5, 1H), 7.38 - 7.29 (m, 1H), 7.22 (s, 1H), 7.21 - 7.15 (m, 2H), 7.09 (ddd, *J* = 7.9, 7.1, 1.0, 1H), 6.90 (d, *J* = 8.5, 1H), 4.38 - 4.21 (m, 4H), 3.92 - 3.81 (m, 1H), 3.72 (dd, *J* = 13.2, 5.3, 1H), 3.15 (ddd, *J* = 15.5, 12.0, 5.6, 1H), 2.93 (dd, *J* = 15.5, 3.7, 1H), 2.28 (s, 3H), 1.34 (t, *J* = 7.1, 3H), 1.25 (t, *J* = 7.1, 3H); ¹³C NMR (101 MHz, CDCl₃) δ 192.1, 169.3, 168.4, 159.1, 153.6, 136.1, 136.0, 135.4, 132.1, 130.4, 127.7, 126.1, 123.0, 119.9, 119.7, 118.3, 118.2, 113.9, 112.0, 108.1, 106.1, 68.2, 63.2, 61.7, 52.4, 23.4, 20.8, 14.5, 14.3; HRMS: Calcd for C₂₉H₂₉O₆N₂ [M+H]⁺: 501.20201 Found: 501.20133.

### Example 4

Preparation of Dimethyl-3-(2-hydroxybenzoyl)-9-methoxy-6,7,12,12b-tetrahydro-indolo[2,3-*a*]quinolizine-1,12b-dicarboxylate (1-04):

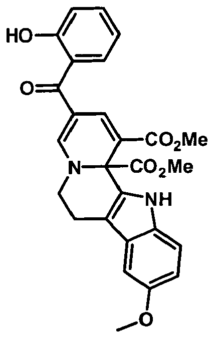

According to the general procedure 1, 0.5 mmol (87 mg) 3-formylchromone was reacted with 0.71 mmol (101 mg) dimethylacetylenedicarboxylate, 0.3 mmol (79 mg) triphenylphosphine, 0.55 mmol (105 mg) 5-methoxytryptamine and 0.75 mmol (174 mg) 1-(*R*)-(-)-camphorsulfonic acid in toluene (5 ml). After purification 0.38 mmol (186 mg, 76% yield) of the desired compound was isolated as a yellow solid; Rf = 0.26 (cyclohexane - ethyl acetate 60:40); mp 163°C (decomposition); 1H NMR (400 MHz, CDCl₃) δ 11.25 (s, 1H), 8.92 (s, 1H), 7.93 - 7.92 (m, 1H), 7.61 (d, J = 1.4, 1H), 7.46 - 7.36 (m, 2H), 7.22 (s, 1H), 7.03 - 6.98 (m, 1H), 6.89 (d, J = 2.4, 1H), 6.88 - 6.81 (m, 2H), 3.85 (s, 3H), 3.83 (s, 3H), 3.81 (s, 3H), 3.80 - 3.67 (m, 2H), 3.12 (ddd, J = 15.6, 12.0, 5.7, 1H), 2.88 (dd, J = 15.4, 4.0, 1H); 13C NMR (126 MHz, CDCl₃) δ 192.1, 170.1, 168.8, 161.5, 154.5, 153.5, 136.3, 134.6, 132.5, 131.4, 130.4, 126.5, 120.0, 118.6, 118.5, 113.6, 113.3, 112.8, 107.9, 106.1, 100.4, 68.1, 56.2, 54.0, 52.7, 52.6, 23.3; HRMS: Calcd for C₂₇H₂₅O₇N₂ [M+H]⁺: 489.16563 Found: 489.16500.
The enantiomers of **1-04** i.e. **(-)-1-04** and **(+)-1-04** were purified by HPLC on an Agilent 1100 using a Daicel IC-column and isohexane / methylene chloride / ethanol (60:40:0.8) as mobile phase. **(-)-1-04:** [α]^{D}₂₀, - 66° (c = 0.299 g/100ml); **(+)-1-04:** [α]^{D}₂₀, + 80° (c = 0.247 g/100ml).

### Example 5

Preparation of Dimethyl-3-(5-bromo-2-hydroxybenzoyl)-9-methoxy-6,7,12,12b-tetrahydro-indolo[2,3-*a*]quinolizine-1,12b-dicarboxylate (1-05):

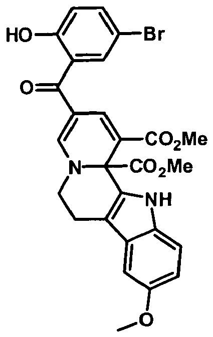

According to the general procedure 1, 0.5 mmol (127 mg) 6-bromo-3-formylchromone was reacted with 0.71 mmol (101 mg) dimethylacetylenedicarboxylate, 0.3 mmol (79 mg) triphenylphosphine, 0.55 mmol (105 mg) 5-methoxytryptamine and 0.75 mmol (174 mg) 1-(*R*)-(-)-camphorsulfonic acid in toluene (5 ml). After purification 0.31 mmol (176 mg, 62% yield) of the desired compound was isolated as a yellow solid; Rf = 0.28 (cyclohexane - ethyl acetate 60:40); mp 197°C (decomposition); ¹H NMR (400 MHz, CDCl₃) δ 11.11 (s, 1H), 8.89 (s, 1H), 7.89 (d, *J* = 1.5, 1H), 7.60 (d, *J* = 1.5, 1H), 7.53 (d, *J* = 2.4, 1H), 7.46 (dd, *J* = 8.8, 2.5, 1H), 7.24 (d, *J* = 8.8, 1H), 6.88 (ddd, *J* = 11.2, 6.3, 1.9, 3H), 3.87 (s, 3H), 3.83 (s, 3H), 3.82 (s, 3H), 3.81 - 3.72 (m, 2H), 3.13 (ddd, *J* = 15.5, 11.8, 5.8, 1H), 2.91 (dd, *J* = 15.5, 3.4, 1H); ¹³C NMR (101 MHz, CDCl₃) δ 190.4, 170.0, 168.7, 160.2, 154.5, 153.6, 137.1, 135.6, 132.4, 132.4, 131.3, 126.4, 121.4, 120.4, 114.1, 113.4, 112.8, 110.3, 107.8, 105.8, 100.2, 68.2, 56.1, 54.1, 52.8, 52.7, 23.4; HRMS: Calcd for C₂₇H₂₄O₇N₂Br [M+H]⁺: 567.07614 Found: 567.07611.

### Example 6

Preparation of Dimethyl-3-(5-chloro-2-hydroxybenzoyl)-6,7,12,12b-tetrahydro-indolo[2,3-*a*]quinolizine-1,12b-dicarboxylate (1-06):

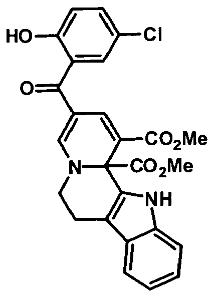

According to the general procedure l, 1.0 mmol (208 mg) 6-chloro-3-formylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(*R*)-(-)-camphorsulfonic acid in toluene (5 ml). After purification 0.39 mmol (190 mg, 39% yield) of the desired compound was isolated as a yellow solid; Rf = 0.38 (cyclohexane - ethyl acetate 60:40); mp 232 °C (decomposition); ¹H NMR (400 MHz, CDCl₃) δ 11.09 (s, 1H), 9.02 (s, 1H), 7.90 (d, *J* = 1.5, 1H), 7.61 (d, *J* = 1.5, 1H), 7.47 (d, *J* = 7.9, 1H), 7.39 (d, *J* = 2.5, 1H), 7.37 - 7.30 (m, 2H), 7.21 - 7.17 (m, 1H), 7.17 - 7.05 (m, 1H), 6.94 (d, *J* = 8.8, 1H), 3.88 (s, 3H), 3.82 (s, 3H), 3.81 - 3.73 (m, 2H), 3.16 (ddd, *J* = 15.6, 11.9, 5.8, 1H), 2.95 (dd*, J* = 15.6, 3.4, 1H); ¹³C NMR (101 MHz, CDCl₃) δ 190.6, 170.0, 168.7, 159.8, 153.6, 136.2, 135.8, 134.3, 131.7, 129.5, 126.0, 123.4, 123.2, 120.8, 120.0, 120.0, 118.4, 114.0, 112.0, 108.2, 105.8, 68.2, 54.1, 52.8, 52.7, 23.4; HRMS: Calcd for C₂₆H₂₂O₆N₂Cl [M+H]**⁺**: 493.11609 Found:493.11565.

### Example 7

Preparation of Dimethyl-3-(5-bromo-2-hydroxybenzoyl)-6,7,12,12b-tetrahydro-indolo[2,3-*a]*quinolizine-1,12b-dicarboxylate (1-07):

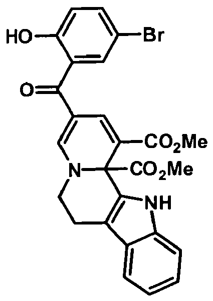

According to the general procedure I, 1.0 mmol (252 mg) 6-bromo-3-formylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(R)-(-)-camphorsulfonic acid in toluene (5 ml). After purification 0.39 mmol (210 mg, 39% yield) of the desired compound was isolated as a yellow solid; Rf = 0.37 (cyclohexane - ethyl acetate 60:40); mp 220°C (decomposition); 1H NMR (400 MHz, CDCl₃) δ 11.11 (s, 1H), 9.02 (s, 1H), 7.90 (d, J = 1.4, 1H), 7.60 (d, J = 1.4, 1H), 7.53 (d, J = 2.4, 1H), 7.50 - 7.42 (m, 2H), 7.35 (d, J = 8.1, 1H), 7.22 - 7.15 (m, 1H), 7.10 (t, J = 7.5, 1H), 6.89 (d, J = 8.8, 1H), 3.88 (s, 3H), 3.82 (s, 3H), 3.81 - 3.72 (m, 2H), 3.25 - 3.08 (m, 1H), 2.95 (dd, J = 15.5, 3.6, 1H); 13C NMR (101 MHz, CDCl₃) δ 190.4, 170.0, 168.7, 160.2, 153.7, 137.1, 136.2, 135.7, 132.4, 131.7, 126.0, 123.2, 121.4, 120.4, 120.0, 118.4, 114.0, 112.0, 110.3, 108.2, 105.8, 68.2, 54.1, 52.8, 52.7, 23.4; HRMS: Calcd for C₂₆H₂₂O₆N₂Br [M+H]⁺: 537.06558 Found: 537.06542.

### Example 8

Preparation of Dimethyl-3-(2-hydroxy-5-methylbenzoyl)-6,7,12,12b-tetrahydro-indolo[2,3-*a*]quinolizine-1,12b-dicarboxylate (1-08):

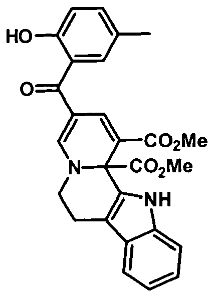

According to the general procedure 1, 1.0 mmol (188 mg) 3-formyl-6-methylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(R)-(-)-camphorsulfonic acid in toluene (5 ml). After purification 0.56 mmol (264 mg, 56% yield) of the desired compound was isolated as a yellow solid; Rf = 0.36 (cyclohexane - ethyl acetate 60:40); mp 197°C; ¹H NMR (400 MHz, CDCl₃) δ 10.99 (s, 1H), 9.03 (s, 1H), 7.93 (d, *J* = 1.4, 1H), 7.60 (d, *J* = 1.3, 1H), 7.47 (d, *J* = 7.8, 1H), 7.35 (d, *J* = 8.1, 1H), 7.23 - 7.17 (m, 3H), 7.10 (dd, *J* = 11.0, 3.9, 1H), 6.94 - 6.85 (m, 1H), 3.86 (s, 3H), 3.81 (s, 3H), 3.79 - 3.68 (m, 2H), 3.15 (ddd, *J* = 15.7, 11.9, 5.7, 1H), 2.93 (dd, *J* = 15.5, 3.6, 1H), 2.28 (s, 3H); ¹³C NMR (101 MHz, CDCl₃) δ 192.1, 170.2, 168.9, 159.1, 153.5, 136.5, 136.2, 135.5, 132.0, 130.4, 127.8, 126.1, 123.1, 120.0, 119.7, 118.4, 118.2, 113.4, 112.0, 108.2, 106.1, 68.1, 54.0, 52.7, 52.5, 23.4, 20.8; HRMS: Calcd for C₂₇H₂₅O₆N₂ [M+H]⁺: 473.17071 Found: 473.17010.

### Example 9

Preparation of Dimethyl-3-(2-hydroxy-5-isopropylbenzoyl)-6,7,12,12b-tetrahydro-indolo[2,3-*a]*quinolizine-1,12b-dicarboxylate (1-09):

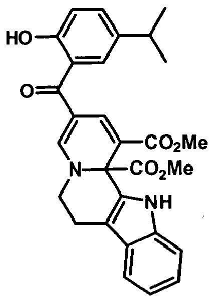

According to the general procedure I, 1.0 mmol (252 mg) 3-formyl-6-isopropylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(*R*)-(-)-camphorsulfonic acid in toluene (5 ml). After purification 0.66 mmol (330 mg, 66% yield) of the desired compound was isolated as a yellow solid; Rf = 0.41 (cyclohexane - ethyl acetate 60:40); ¹H NMR (400 MHz, CDCl₃) δ 10.99 (s, 1H), 9.04 (s, 1H), 7.94 (d, *J* = 1.3, 1H), 7.66 (d, *J* = 1.2, 1H), 7.46 (d, *J* = 7.8, 1H), 7.33 (d, *J* = 8.1, 1H), 7.27 - 7.21 (m, *J* = 14.6, 2H), 7.20 - 7.15 (m, 1H), 7.09 (dd, *J* = 11.1, 3.8, 1H), 6.90 (d, *J* = 9.1, 1H), 3.82 (s, 3H), 3.85 - 3.76 (m, *J̅* = 13.2, 1H), 3.79 (s, 3H), 3.70 (dd, J = 13.1, 5.3, 1H), 3.15 (ddd, J = 17.5, 12.0, 5.6, 1H), 2.93 (dd, *J* = 15.5, 3.8, 1H), 2.89 - 2.77 (m, 1H), 1.20 (d, *J* = 6.9, 7H); ¹³C NMR (101 MHz, CDCl₃) δ 192.1, 170.1, 168.8, 159.2, 153.7, 138.9, 136.9, 136.1, 133.1, 132.0, 127.9, 126.1, 123.1, 120.0, 119.6, 118.4, 118.1, 112.9, 112.0, 108.2, 105.9, 68.1, 54.0, 52.6, 33.4, 27.1, 24.3, 24.3, 23.4; HRMS: Calcd for C₂₉H₂₉O₆N₂ [M+H]⁺: 501.20201 Found: 501.20134.

### Example 10

Preparation of Dimethyl-3-(3,5-dichloro-2-hydroxybenzoyl)-6,7,12,12b-tetrahydro-indolo[2,3*-a*]quinolizine-1,12b-dicarboxylate (1-10):

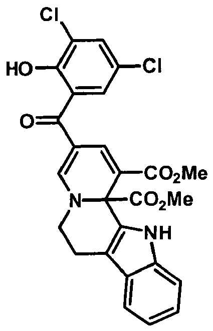

According to the general procedure I, 1.0 mmol (242 mg) 6,8-dichloro-3-formylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(*R*)-(-)-camphorsulfonic acid in toluene (5 ml). After purification 0.20 mmol (105 mg, 20% yield) of the desired compound was isolated as a yellow solid; Rf = 0.40 (cyclohexane - ethyl acetate 60:40); mp 238.2°C (decomposition); ¹H NMR (400 MHz, CDCl₃) δ 11.59 (s, 1H), 9.01 (s, 1H), 7.88 (d, *J* = 1.5, 1H), 7.63 (d, *J* = 1.5, 1H), 7.53 - 7.43 (m, *J* = 7.1, 1.5, 2H), 7.34 (d, *J* = 8.1, 1H), 7.32 (d, *J* = 2.5, 1H), 7.20 (ddd, *J* = 8.2, 7.1, 1.2, 1H), 7.10 (ddd, *J* = 7.9, 7.1, 1.0, 1H), 3.89 (s, 3H), 3.83 (d, *J* = 2.6, 3H), 3.81 (dd, *J* = 13.2, 5.2, 2H), 3.16 (ddd, *J* = 15.6, 11.9, 5.8, 1H), 2.96 (dd, *J* = 15.6, 3.6, 1H); ¹³C NMR (101 MHz, CDCl₃) δ 189.8, 169.9, 168.6, 155.7, 153.9, 136.2, 135.4, 134.0, 131.5, 128.0, 126.0, 124.0, 123.3, 123.2, 121.5, 120.1, 118.4, 114.2, 112.0, 108.2, 105.6, 68.3, 54.2, 52.9, 52.9, 23.4; HRMS: Calcd for C₂₆H₂₁O₇N₂l₂ [M+H]⁺: 527.07712 Found: 527.07684.

### Example 11

Preparation of Dimethyl-3-(3,5-dibromo-2-hydroxybenzoyl)-6,7,12,12b-tetrahydro-indolo[2,3-*a*]quinolizine-1,12b-dicarboxylate (1-11):

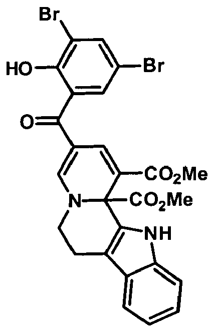

According to the general procedure 1, 1.0 mmol (331 mg) 6,8-dibromo-3-formylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-(*R*)-(-)-camphorsulfonic acid in toluene (5 ml). After purification 0.20 mmol (123 mg, 20% yield) of the desired compound was isolated as a yellow solid; Rf = 0.40 (cyclohexane - ethyl acetate 60:40); mp 131°C; ¹H NMR (400 MHz, CDCl₃) δ 11.75 (s, 1H), *9.01 (s, 1H),* 7.88 (d*, J =* 1.6, 1H), 7.77 (d, *J* = 2.3, 1H), 7.63 (d, *J* = 1.5, 1H)*,* 7.49 (d, *J =* 2.3, 1H)*,* 7.47 (dd, *J* = 7.9, 0.6, 1H), 7.34 (d, *J* = 8.1, 1H), 7.20 (ddd, *J* = 8.2, 7.1, 1.2, 1H), 7.10 (ddd, *J* = 8.0, 7.1, 1.0, 1H), 3.88 (s, 3H), 3.82 (d, *J* = 2.8, 3H), 3.80 - 3.70 (m, 2H), 3.16 (ddd*, J* = 15.6, 11.8, 5.8, 1H), 2.96 (dd, *J* = 15.6, 3.5, 1H); 13C NMR (101 MHz, CDCl₃) δ 189.7, 169.9, 168.6, 157.0, 153.9, 139.5, 136.2, 135.4, 131.6, 131.5, 126.0, 123.3, 121.9, 120.1, 118.4, 114.2, 113.2, 112.0, 110.3, 108.2, 105.5, 68.3, 54.2, 52.9, 52.9, 23.4; HRMS: Calcd for C₂₆H₂₁O₆N₂Br₂ [M+H]⁺: 614.97609 Found: 614.97638.

### Example 12

Preparation of Dimethyl-3-(5-chloro-2-hydroxy-4-methylbenzoyl)-6,7,12,12b-tetrahydro-indolo[2,3-*a*]quinolizine-1,12b-dicarboxylate (1-12):

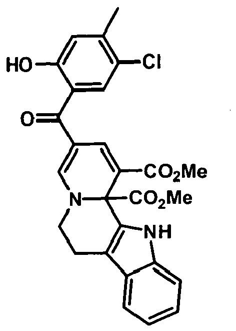

According to the general procedure I, 1.0 mmol (222 mg) 6-chloro-3-formyl-7-methylchromone was reacted with 1.3 mmol (184 mg) dimethylacetylenedicarboxylate, 0.6 mmol (157 mg) triphenylphosphine, 1.1 mmol (176 mg) tryptamine and 1.1 mmol (255 mg) 1-*(R*)-(-)-camphorsulfonic acid in toluene (5 ml). After purification 0.60 mmol (305 mg, 60% yield) of the desired compound was isolated as a yellow solid; Rf = 0.35 (cyclohexane - ethyl acetate 60:40); mp °C; ¹H NMR (400 MHz, CDCl₃) δ 11.24 (s, 1H), 9.06 (s, 1H), 7.94 (d, *J* = 1.4, 1H), 7.63 (d, *J* = 1.3, 1H), 7.52 (d*, J* = 7.8, 1H), 7.43 (s, 1H), 7.39 (d**, *J*** = 8.1, 1H), 7.27 - 7.19 (m, 1H), 7.18 - 7.10 (m, 1H), 6.92 (s, 1H), 3.92 (s, 3H), 3.86 (s, 3H), 3.85 - 3.75 (m, 2H), 3.20 (ddd, *J =* 15.8, 11.9, 5.8, 1H), 2.98 (dd, *J =* 15.5, 3.6, 1H), 2.39 (s, 3H); ¹³C NMR (101 MHz, CDCl₃) δ 190.6, 170.1, 168.8, 159.9, 153.4, 143.4, 136.2, 135.9, 131.8, 130.0, 126.1, 123.9, 123.1, 120.5, 120.0, 118.8, 118.4, 113.8, 112.0, 108.2, 105.8, 68.1, 54.1, 52.8, 52.6, 23.4, 20.8. HRMS: Calcd for C₂₇H₂₄O₆N₂Cl [M+H]⁺: 507.13174 Found: 507.13130.

### Example 13

Preparation of Diethyl-3-(2-hydroxybenzoyl)-9-methoxy-6,7,12,12b-tetrahydro-indolo[2,3-*a*]quinolizine-1,12b-dicarboxylate (1-13):

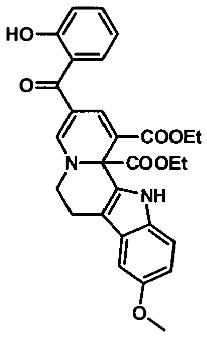

According to the general procedure I, 1.0 mmol (174 mg) 3-formylchromone was reacted with 1.3 mmol (221 mg) diethylacetylenedicarboxylate, 0.6 mmol (157 mg) triphenylphosphine, 1.1 mmol (209 mg) 5-methoxytryptamine and 1.5 mmol (348 mg) 1-(*R*)-(-)-camphorsulfonic acid in toluene (10 ml). After purification 0.76 mmol (392 mg, 76% yield) of the desired compound was isolated as a yellow solid; Rf = 0.55 (cyclohexane - ethyl acetate 60:40); mp 212°C; ¹H NMR (400 MHz, cdcl₃) δ 11.25 (s, 1H), 8.98 (s, 1H), 7.92 (d, *J* = 1.5, 1H), 7.59 (d, *J* = 1.5, 1H), 7.43 (dd, *J* = 7.8, 1.6, 1H), 7.39 (ddd, *J* = 8.4, 7.3, 1.7, 1H), 7.22 (dd, *J* = 8.8, 0.5, 1H), 7.00 (dd, *J* = 8.4, 1.0, 1H), 6.89 (d, *J* = 2.4, 1H), 6.87 - 6.82 (m, 2H), 4.37 - 4.20 (m, 4H), 3.90 - 3.78 (m, 1H), 3.83 (s, 3H), 3.71 (dd, *J* = 13.2, 5.0, 1H), 3.11 (ddd, *J* = 15.5, 12.0, 5.7, 1H), 2.88 (dd, *J* = 15.4, 3.5, 1H), 1.34 (t, *J* = 7.1, 3H), 1.25 (t, *J* = 7.1, 3H); ¹³C NMR (101 MHz, cdcl₃) δ 192.1, 169.3, 168.3, 161.4, 154.4, 153.5, 135.7, 134.5, 132.7, 131.3, 130.4, 126.5, 120.1, 118.6, 118.4, 114.2, 113.1, 112.8, 107.9, 106.1, 100.3, 68.2, 63.21, 61.7, 56.1, 52.4, 23.4, 14.5, 14.3; HRMS: Calcd for C₂₉H₂₉O₇N₂ [M+H]⁺: 517.19693, Found: 517.19623.

### Example 14

Preparation of Diethyl-3-(5-bromo-2-hydroxybenzoyl)-6,7,12,12b-tetrahydro-indolo[2,3-*a*]quinolizine-1,12b-dicarboxylate (1-14):

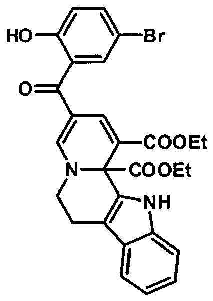

According to the general procedure I, 1.0 mmol (253 mg) 6-bromo-3-formylchromone was reacted with 1.3 mmol (221 mg) diethylacetylenedicarboxylate, 0.6 mmol (157 mg) triphenylphosphine, 1.1 mmol (176 mg) tryptamine and 1.5 mmol (348 mg) 1-(R)-(-)-camphorsulfonic acid in toluene (10 ml). After purification 0.53 mmol (297 mg, 52% yield) of the desired compound was isolated as a yellow solid; Rf = 0.71 (cyclohexane - ethyl acetate 60:40); mp 215°C;
¹H NMR (400 MHz, cdcl₃) δ 11.16 (s, 1H), 9.09 (s, 1H), 7.91 (d, *J* = 1.6, 1H), 7.62 (d, *J* = 1.6, 1H), 7.54 (d, *J* = 2.5, 1H), 7.49 - 7.44 (m, 2H), 7.34 (dt, *J* = 8.2, 0.9, 1H), 7.19 (ddd, *J* = 8.2, 7.1, 1.2, 1H), 7.09 (ddd, *J* = 7.9, 7.1, 1.0, 1H), 6.89 (d, *J* = 8.8, 1H), 4.38 - 4.24 (m, 4H), 3.93 - 3.83 (m, 1H), 3.76 (dd, *J* = 13.5, 5.4, 1H), 3.22 - 3.10 (m, 1H), 2.95 (dd, *J* = 15.6, 3.6, 1H), 1.36 (t, *J* = 7.1, 3H), 1.26 (t, *J* = 7.1, 3H);
¹³C NMR (101 MHz, cdcl₃) δ 190.4, 169.1, 168.2, 160.3, 153.7, 137.02, 136.2, 135.4, 132.5, 131.8, 126.1, 123.1, 121.5, 120.4, 120.0, 118.4, 114.5, 112.0, 110.2, 108.2, 105.8, 68.4, 63.3, 61.8, 52.6, 23.4, 14.4, 14.3; HRMS: Calcd for C₂₈H₂₉O₆N₂Br [M+H]⁺: 565.09688, Found: 565.09666.

### Example 15

Preparation of Diethyl-3-(3,5-dichloro-2-hydroxybenzoyl)-6,7,12,12b-tetrahydro-indolo[2,3-*a*]quinolizine-1,12b-dicarboxylate (1-15):

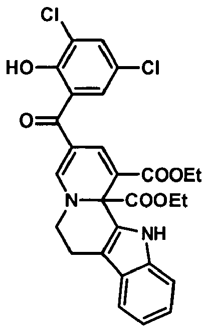

According to the general procedure 1, 1.0 mmol (243 mg) 6,8-dichloro-3-formylchromone was reacted with 1.3 mmol (221 mg) diethylacetylenedicarboxylate, 0.6 mmol (157 mg) triphenylphosphine, 1.1 mmol (176 .mg) tryptamine and 1.5 mmol (348 mg) 1-(*R*)-(-)-camphorsulfonic acid in toluene (10 ml). After purification 0.20 mmol (112 mg, 20% yield) of the desired compound was isolated as an amorphous yellow solid; Rf = 0.72 (cyclohexane - ethyl acetate 60:40); ¹H NMR (400 MHz, cdcl₃) δ 11.65 (s, 1H), 9.09 (s, 1H), 7.88 (d, *J* = 1.6, 1H), 7.65 (d, *J* = 1.5, 1H), 7.50 - 7.44 (m, 2H), 7.37 - 7.31 (m, *J* = 5.4, 2H), 7.19 (ddd, *J* = 8.2, 7.1, 1.2, 1H), 7.09 (ddd, *J* = 8.0, 7.1, 1.0, 1H), 4.42 - 4.20 (m, 4H), 3.95 - 3.82 (m, 1H), 3.77 (dd, *J* = 13.2, 5.0, 1H), 3.16 (ddd, *J =* 17.6, 12.0, 5.6, 1H), 2.96 (dd, *J* = 15.6, 3.6, 1H), 1.35 (t, *J* = 7.1, 3H), 1.25 (t, *J* = 7.1, 3H); ¹³C NMR (101 MHz, cdcl₃) δ 189.8, 169.0, 168.1, 155.7, 154.0, 136.2, 135.1, 133.9, 131.6, 128.0, 126.1, 124.0, 123.2, 123.1, 121.6, 120.0, 118.4, 114.7, 112.0, 108.2, 105.6, 68.4, 63.4, 61.9, 52.8, 23.4, 14.4, 14.2; HRMS: Calcd for C₂₈H₂₅O₆N₂Cl₂ [M+H]⁺: 555.10842, Found: 555.10811.

### Example 16

Preparation of Diethyl-3-(2-hydroxy-5-methylbenzoyl)-9-methoxy-6,7,12,12b-tetrahydroindolo[2,3-*a*]quinolizine-1,12b-dicarboxylate (1-16):

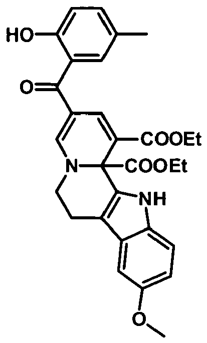

According to the general procedurel, 1.0 mmol (188 mg) 3-formyl-6-methylchromone was reacted with 1.3 mmol (221 mg) diethylacetylenedicarboxylate, 0.6 mmol (157 mg) triphenylphosphine, 1.1 mmol (209 mg) 5-methoxytryptamine and 1.5 mmol (348 mg) 1-(*R*)-(-)-camphorsulfonic acid in toluene (10 ml). After purification 0.44 mmol (236 mg, 45% yield) of the desired compound was isolated as a yellow solid; Rf = 0.56 (cyclohexane - ethyl acetate 60:40); mp 228°C; ¹H NMR (400 MHz, cdcl₃) δ 11.01 (s, 1H), 8.97 (s, 1H), 7.92 (d, *J* = 1.5, 1H), 7.60 (d, *J* = 1.5, 1H), 7.24 - 7.19 (m, 3H), 6.92 - 6.88 (m, 2H), 6.84 (dd, *J* = 8.8, 2.5, 1H), 4.34 - 4.23 (m, 4H), 3.89 - 3.80 (m, 1H), 3.83 (s, 3H), 3.71 (dd, *J* = 13.3,5.1, 1H), 3.11 (ddd, *J* = 15.5, 12.1, 5.6, 1H), 2.89 (dd, *J* = 15.5, 3.8, 1H), 2.27 (s, 3H), 1.34 (t, *J* = 7.1, 3H), 1.25 (t, *J* = 7.1, 3H); ¹³C NMR (101 MHz, cdcl₃) δ 192.1, 169.3, 168.4, 159.2, 154.4, 153.5, 136.0, 135.4, 132.8, 131.3, 130.4, 127.7, 126.5, 119.8, 118.2, 113.9, 113.1, 112.8, 107.8, 106.1, 100.3, 68.3, 63.2, 61.7, 56.1, 52.4, 23.4, 20.8, 14.5, 14.3; HRMS: Calcd for C₃₀H₃₁O₇N₂ [M+H]⁺: 531.21258, Found: 531.21189.

### Example 17

Preparation of Diethyl-3-(2-hydroxy-5-isopropylbenzoyl)-9-methoxy-6,7,12,12b-tetrahydroindolo[2,3*-a*]quinolizine-1,12b-dicarboxytate (1-17):

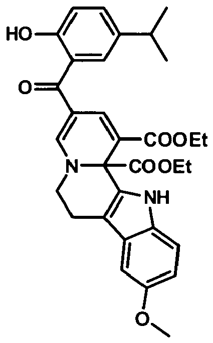

According to the general procedure I, 1.0 mmol (216 mg) 3-formyl-6-isopropylchromone was reacted with 1.3 mmol (221 1 mg) diethylacetylenedicarboxylate, 0.6 mmol (157 mg) triphenylphosphine, 1.1 mmol (209 mg) 5-methoxytryptamine and 1.5 mmol (348 mg) 1-(*R*)-(-)-camphorsulfonic acid in toluene (10 ml). After purification 0.65 mmol (361 mg, 65% yield) of the desired compound was isolated as an amorphous yellow solid; Rf = 0.63 (cyclohexane - ethyl acetate 60:40); ¹H NMR (400 MHz, cdcl₃) δ 11.05 (s, 1H), 8.95 (s, 1H), 7.89 (d, *J* = 1.5, 1H), 7.65 (d, *J* = 1.5, 1H), 7.21 (ddd, *J* = 8.8, 7.3, 3.1, 3H), 6.91 - 6.85 (m, 2H), 6.82 (dd, *J* = 8.8, 2.5, 1H), 4.33 - 4.17 (m, 4H), 3.86 - 3.78 (m, 1H), 3.80 (s, 3H), 3.67 (dd, *J* = 13.2, 5.1, 1H), 3.10 (ddd, *J* = 15.3, 12.0, 5.6, 1H), 2.87 (dd, *J* = 15.8, 4.0, 1H), 1.28 (t, *J* = 7.1, 3H), 1.23 - 1.17 (m, 9H); ¹³C NMR (101 MHz, cdcl₃) δ 192.1, 169.2, 168.4, 159.3, 154.4, 153.6, 138.8, 136.2, 133.0, 132.9, 131.3, 127.9, 126.5, 119.7, 118.1, 113.5, 113.2, 112.8, 107.9, 105.9, 100.3, 68.3, 63.2, 61.7, 56.1, 52.4, 33.4, 24.4, 24.3, 23.4, 14.5, 14.3; HRMS: Calcd for C₃₂H₃₅O₇N₂ [M+H]⁺: 559.24388, Found: 559.24326.

### Example 18

Preparation of Diethyl-3-(5-chloro-2-hydroxy-4-methylbenzoyl)-6,7,12,12b-tetrahydroindolo[2,3-*a*]quinolizine-1,12b-dicarboxylate (1-18):

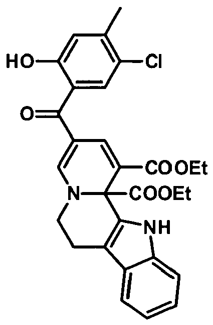

According to the general procedure I, 1.0 mmol (223 mg) 6-chloro-3-formyl-7-methylchromone was reacted with 1.3 mmol (221 mg) diethylacetylenedicarboxylate, 0.6 mmol (157 mg) triphenylphosphine, 1.1 mmol (176 mg) tryptamine and 1.5 mmol (348 mg) 1-(R)-(-)-camphorsulfonic acid in toluene (10 ml). After purification 0.40 mmol (216 mg, 40% yield) of the desired compound was isolated as a yellow solid; Rf = 0.74 (cyclohexane - ethyl acetate 60:40); mp 196°C; ¹H NMR (400 MHz, cdcl₃) δ 11.24 (s, 1H), 9.10 (s, 1H), 7.90 (d, *J* = 1.5, 1H), 7.60 (d, *J* = 1.5, 1H), 7.47 (d, *J* = 7.7, 1H), 7.41 (s, 1H), 7.34 (d, *J* = 8.1, 1H), 7.19 (ddd, *J* = 8.2, 7.1, 1.2, 1H), 7.14 - 7.06 (m, 1H), 6.88 (d, *J* = 0.7, 1H), 4.38 - 4.22 (m, 4H), 3.93 - 3.80 (m, 1H), 3.74 (dd, *J* = 13.2, 4.9, 1H), 3.24 - 3.07 (m, 1H), 2.94 (dd, *J* = 15.5, 3.5, 1H), 2.35 (s, 3H), 1.35 (t, *J* = 7.1, 3H), 1.26 (t, *J* = 7.1, 3H); ¹³C NMR (101 MHz, cdcl₃) δ 190.5, 169.2, 168.3, 159.9, 153.4, 143.3, 136.2, 135.5, 131.9, 130.0, 126.1, 123.8, 123.0, 120.5, 119.9, 118.9, 118.3, 114.3, 112.0, 108.2, 105.8, 68.3, 63.3, 61.8, 52.5, 23.4, 20.7, 14.4, 14.3; HRMS: Calcd for C₂₉H₂₈O₆N₂Cl [M+H]⁺: 535.16304, Found: 535.16262.

### Example 19

Preparation of Diethyl-3-(5-chloro-2-hydroxybenzoyl)-6,7,12,12b-tetrahydro-indolo[2,3-*a*]quinolizine-1,12b-dicarboxylate (1-19):

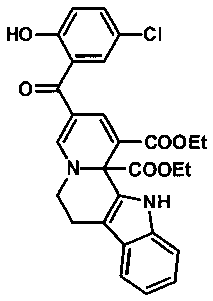

According to the general procedure I, 1.0 mmol (209 mg) 6-chloro-3-formylchromone was reacted with 1.3 mmol (221 mg) diethylacetylenedicarboxylate, 0.6 mmol (157 mg) triphenylphosphine, 1.1 mmol (176 mg) tryptamine and 1.5 mmol (348 mg) 1-(*R*)-(-)-camphorsulfonic acid in toluene (10 ml). After purification 0.65 mmol (337 mg, 65% yield) of the desired compound was isolated as a yellow solid; Rf = 0.71 (cyclohexane - ethyl acetate 60:40); mp 216°C; ¹H NMR (400 MHz, cdcl₃) δ 11.13 (s, 1H), 9.09 (s, 1H), 7.91 (d, *J* = 1.6, 1H), 7.61 (d, *J* = 1.5, 1H), 7.47 (d, *J* = 7.9, 1H), 7.40 (d, *J* = 2.6, 1H), 7.35 - 7.34 (m, 1H), 7.32 (dd, *J* = 1.6, 0.7, 1H), 7.19 (ddd, *J* = 8.2, 7.1, 1.2, 1H), 7.09 (ddd, *J* = 8.0, 7.1, 1.0, 1H), 6.94 (d, *J* = 8.8, 1H), 4.42 - 4.21 (m, 4H), 3.95 - 3.81 (m, 1H), 3.76 (dd, *J* = 13.0, 5.2, 1H), 3.16 (ddd, *J* = 15.5, 12.1, 5.7, 1H), 2.95 (dd, *J* = 15.6, 3.5, 1H), 1.35 (t, *J* = 7.1, 3H), 1.26 (t, *J* = 7.1, 3H); 13C NMR (101 MHz, cdcl₃) δ 190.5, 169.1, 168.2, 159.8, 153.7, 136.2, 135.4, 134.2, 131.8, 129.5, 126.1, 123.3, 123.0, 120.9, 119.9 (2 C), 118.4, 114.5, 112.0, 108.2, 105.8, 68.3, 63.32, 61.8, 52.6, 23.4, 14.4, 14.3; HRMS: Calcd for C₂₈H₂₆ON₂Cl [M+H]⁺: 521.14739, Found: 521.14689.

### Example 20

Preparation of Diethyl-3-(5-chloro-2-hydroxybenzoyl)-9-methoxy-6,7,12,12b-tetrahydroindolo[2,3-*a*]quinolizine-1,12b-dicarboxylate (1-20):

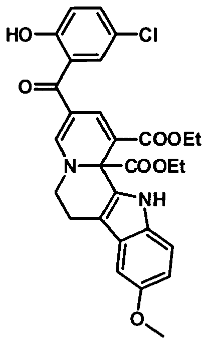

According to the general procedure I, 1.0 mmol (209 mg) 6-chloro-3-formylchromone was reacted with 1.3 mmol (221 mg) diethylacetylenedicarboxylate, 0.6 mmol (157 mg) triphenylphosphine, 1.1 mmol (209 mg) 5-methoxytryptamine and 1.5 mmol (348 mg) 1-(*R*)-(-)-camphorsulfonic acid in toluene (10 ml). After purification 0.63 mmol (345 mg, 63% yield) of the desired compound was isolated as a yellow solid; Rf = 0.62 (cyclohexane - ethyl acetate 60:40); mp 230°C; ¹H NMR (400 MHz, cdcl₃) δ 11.13 (s, 1H), 8.97 (s, 1H), 7.90 (d, *J* = 1.6, 1H), 7.61 (d*, J* = 1.5, 1H), 7.40 (d, *J* = 2.6, 1H), 7.33 (dd, J = 8.8, 2.6, 1H), 7.22 (d, *J* = 8.8, 1H), 6.94 (d, *J* = 8.8, 1H), 6.89 (d, *J* = 2.4, 1H), 6.85 (dd, *J* = 8.8, 2.5, 1H), 4.39 - 4.21 (m, 4H), 3.93 - 3.80 (m, 1H), 3.83 (s, 3H), 3.75 (dd, *J* = 13.1, 5.3, 1H), 3.13 (ddd, *J* = 15.5, 12.0, 5.7, 1H), 2.91 (dd, *J* = 15.5, 3.6, 1H), 1.35 (t, *J* = 7.1, 3H), 1.25 (t, *J* = 7.1, 3H); ¹³C NMR (101 MHz, cdcl₃) δ 190.5, 169.1, 168.2, 159.8, 154.5, 153.6, 135.3, 134.2, 132.5, 131.4, 129.6, 126.5, 123.3, 120.9, 120.0, 114.6, 113.3, 112.8, 107.8, 105.8, 100.3, 68.4, 63.3, 61.8, 56.1, 52.6, 23.5, 14.4, 14.3; HRMS: Calcd for C₂₉H₂₈O₇N₂Cl [M+H]⁺: 551.15796, Found: 551.15757.

### Example 21

Preparation of Dimethyl-3-(1-hydroxy-2-naphthoyl)-6,7,12,12b-tetrahydroindolo-[2,3-*a*]quinolizine-1,12b-dicarboxylate (1-21):

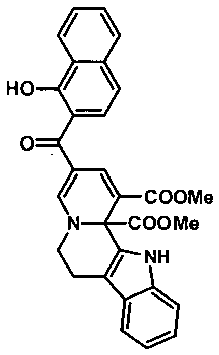

According to the general procedure I, 1.0 mmol (224 mg) 3-formylchromone (4-oxo-4*H*-benzo[g]chromene-3-carbaldehyde) was reacted with 1.3 mmol (185 mg) dimethylacetylenedicarboxylate, 0.6 mmol (157 mg) triphenylphosphine, 1.1 mmol (176 mg) tryptamine and 1.5 mmol (348 mg) 1-(*R*)-(-)-camphorsulfonic acid in toluene (10 ml). After purification 0.20 mmol (100 mg, 52% yield) of the desired compound was isolated as a yellow amorphous solid; Rf = 0.57 (cyclohexane - ethyl acetate 60:40); ¹H NMR (400 MHz, cdcl₃) δ 13.19 (s, 1H), 9.01 (s, 1H), 8.36 (dd, *J* = 8.3, 1.3, 1H), 7.91 (d, *J* = 1.5, 1H), 7.69 (d, *J* = 8.1, 1H), 7.61 (d, *J* = 1.5, 1H), 7.53 (ddd, *J* = 8.2, 6.9, 1.3, 1H), 7.47 - 7.37 (m, 3H), 7.29 (dt, *J* = 8.2, 0.9, 1H), 7.19 (d, *J* = 8.5, 2H), 7.14 (ddd, *J* = 8.2, 7.1, 1.2, 1H), 7.04 (ddd, *J* = 8.0, 7.1, 1.0, 1H), 3.80 (s, 3H), 3.79 - 3.76 (m, 1H), 3.76 (s, 3H), 3.69 (dd, *J* = 13.3, 4.9, 1H), 3.11 (ddd, *J* = 15.6, 11.9, 5.7, 1H), 2.88 (dd, *J* = 15.5, 3.4, 1H); ¹³C NMR (101 MHz, cdcl₃) δ 192.4, 170.2, 168.8, 161.5, 153.3, 136.7, 136.7, 136.2, 131.9, 129.6, 127.5, 126.1, 126.0, 125.7, 125.7, 124.3, 123.1, 119.9, 118.4, 117.8, 113.1, 112.8, 112.0, 108.3, 106.1, 68.1, 53.9, 52.7, 52.5, 23.3; HRMS: Calcd for C₃₀H₂₄O₆N₂ [M+H]⁺: 509.17071, Found: 509.17005.

### Example 22

Preparation of Diethyl-3-(5-bromo-2-hydroxybenzoyl)-9-methoxy-6,7,12,12b-tetrahydroindolo[2,3-*a*]quinolizine-1,12b-dicarboxylate (1-22):

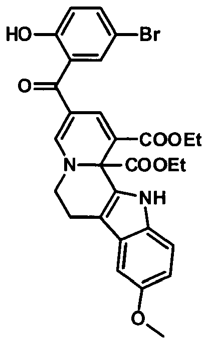

According to the general procedure I, 1.0 mmol (253 mg) 6-bromo-3-formylchromone was reacted with 1.3 mmol (221 mg) diethylacetylenedicarboxylate, 0.6 mmol (157 mg) triphenylphosphine, 1.1 mmol (209 mg) 5-methoxytryptamine and 1.5 mmol (348 mg) 1-(*R*)-(-)-camphorsulfonic acid in toluene (10 ml). After purification 0.54 mmol (320 mg, 54% yield) of the desired compound was isolated as a yellow solid; Rf = 0.60 (cyclohexane - ethyl acetate 60:40); mp 225°C; ¹H NMR (400 MHz, cdcl₃) δ 11.16 (s, 1H), 8.97 (s, 1H), 7.90 (d, *J* = 1.5, 1H), 7.62 (d, *J* = 1.5, 1H), 7.54 (d, *J* = 2.5, 1H), 7.47 (dd, *J* = 8.8, 2.5, 1H), 7.24 - 7.22 (m, 1H), 6.92 - 6.87 (m, 2H), 6.85 (dd, *J* = 8.8, 2.5, 1H), 4.40 - 4.20 (m, 4H), 3.92 - 3.80 (m, 1H), 3.83 (s, 3H), 3.75 (dd, *J* = 13.1, 5.1, 1H), 3.13 (ddd, *J* = 15.5, 12.0, 5.6, 1H), 2.91 (dd, *J* = 15.4, 3.7, 1H), 1.35 (t, *J* = 7.1, 3H), 1.25 (t, *J* = 7.1, 3H); ¹³C NMR (101 MHz, cdcl₃) δ 190.4, 169.1, 168.2, 160.3, 154.5, 153.7, 137.0, 135.3, 132.5, 132.5, 131.4, 126.5, 121.5, 120.4, 114.5, 113.3, 112.8, 110.2, 107.8, 105.8, 100.3, 77.5, 77.2, 76.9, 68.4, 63.3, 61.9, 56.1, 52.7, 23.5, 14.5, 14.3; HRMS: Calcd for C₂₉H₂₈O₇N₂Br [M+H]⁺: 595.10744, **Found: 595.10736.**

### Example 23

Preparation of Dimethyl 3-(4-(benzyloxy)-2-hydroxy-3-methylbenzoyl)-6,7,12,12b-tetrahydroindolo[2,3*-a*]quinolizine-1,12b-dicarboxylate (1-23):

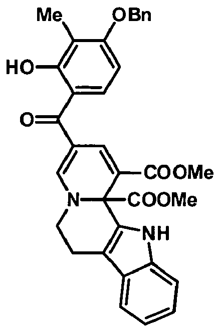

0.14 mmol (40 mg) 7-benzyloxy-8-methyl-3-formylchromone was reacted with 0.18 mmol (25.1 mg) diethylacetylenedicarboxylate, 0.08 mmol (21.38 mg) triphenylphosphine (30 minutes in this case) 0.15 mmol (23.95 mg) tryptamine and 0.15 mmol (34.73 mg) 1-(*R*)-(-)-camphorsulfonic acid in toluene (5 ml). After purification 0.15 mmol (46.3 mg, 58.9 % yield) of the desired compound was isolated as a yellow solid; R_{f} = 0.44 (cyclohexane - ethyl acetate 6:4); yellow solid; mp 216°C; ¹H NMR (400 MHz, CDCl₃): δ 12.03 (s, 1H), 9.07 (s, 1H), 7.90 (d, *J* = 1.5 Hz, 1H), 7.55 (d, *J* = 1.5 Hz, 1H), 7.48 (d, *J* =7.5 Hz, 1H), 7.43-7.39 (m, 4H), 7.36-7.34 (m, 2H), 7.31 (d, *J* = 8.7 Hz, 1H), 7.20 (t, *J* =7.5 Hz, 1H), 7.10 (t, *J* =7.5 Hz, 1H), 6.46 (d, *J* =7.5 Hz, 1H), 5.15 (s, 2H), 3.86 (s, 3H), 3.81 (s, 3H), 3.79-3.60 (m, 2H), 3.19-3-11 (m, 1H) 2.95-2-90 (m, 1H), 2.19 (s, 3H).; ¹³C NMR (100 MHz, CDCl₃): δ 191.7,169.8, 168.3, 161.2, 160.8, 153.2, 136.1, 135.5, 134.3, 131.6, 130.2, 129.1, 128.3, 127.4, 126.2, 124.4, 119.7, 118.4, 118.2, 117.8, 113.4, 111.9, 107.7, 106.2, 71.3, 67.8, 53.7, 52.5, 52.3, 23.1, 21.4, 9.8.; Calculated for C₃₄H₃₁O₇N₂ [M+H⁺]: 579.21258, Found: 579.21225.

### Example 24

Preparation of Dimethyl 3-(2-methoxybenzoyl)-6,7,12,12b-tetrahydroindolo[2,3-*a*]quinolizine-1,12b-dicarboxylate (24):

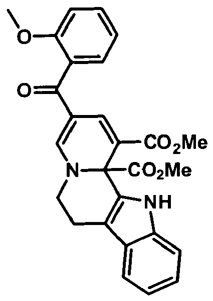

To a solution of 0.65 mmol (300 mg) **7a** in acetone (15 ml) was added 12.9 mmol (1.79 g) potassium carbonate and 3.9 mmol (557 mg) methyl iodide. The mixture was refluxed overnight. TLC (CH/EA 40:60) showed no more starting material. The mixture was poured into saturated ammonium acetate solution (50 ml) and extracted with ethyl acetate (2x 50 ml). The combined organic layers were dried over magnesium sulfate and narrowed under reduced pressure. After purification 0.49 mmol (233 mg, 75% yield) of the desired product was obtained as yellow solid; Rf = 0.14 (cyclohexane - ethyl acetate 60:40); mp 261°C (decomposition); ¹H NMR (400 MHz, cdcl₃) δ 9.05 (s, 1H), 7.93 (s, 1H), 7.45 (d, *J* = 6.9, 1H), 7.40 - 7.30 (m, 3H), 7.22 (dd, *J* = 7.5, 1.7, 1H), 7.17 (ddd, *J* = 8.2, 7.1, 1.2, 1H), 7.11 - 7.05 (m, 1H), 6.98 (td, *J* = 7.4, 0.9, 1H), 6.93 (d, *J* = 8.4, 1H), 3.83 (s, 3H), 3.78 (s, 3H), 3.77 (s, 3H), 3.71 (dd, *J* = 12.0, 4.3, 1H), 3.60 (dd, *J* = 13.1, 5.1, 1H), 3.08 (ddd, *J* = 17.6, 12.0, 5.6, 1H), 2.86 (dd, *J* = 15.4, 3.6, 1H); ¹³C NMR (101 MHz, CDCl₃) δ 188.9, 170.3, 169.2, 156.6, 153.9, 136.1, 132.3, 131.2, 129.2, 128.8, 126.1, 122.9, 120.8, 119.8, 118.3, 112.0, 111.6, 108.0, 107.8, 68.1, 55.9, 53.9, 52.5, 52.2, 23.3; HRMS: Calcd for C₂₇H₂₅O₆N₂ [M+H]⁺: 473.17071, Found: 473.17020.

### Example 25

Characterization of Dimethyl-3-(2-hydroxybenzoyl)-9-hydroxy-6,7,12,12b-tetrahydroindolo[2,3-*a*]quinolizine-1,12b-dicarboxylate (1-25): 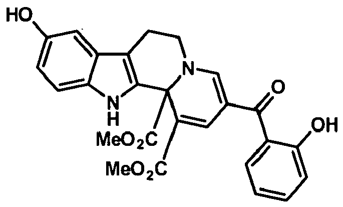 (GP IA) 67% yield; Yellow solid; m.p.: 180 °C (decomposition); TLC (CH/EA, 3:2 v/v): *R*_{F} = 0.25 ; ¹H NMR (400 MHz, CDCl₃) δ 8.87 (s, 1H), 7.93 (s, 1H), 7.62 (s, 1H), 7.50 - 7.35 (m, 2H), 7.16 (d, *J* = 8.7 Hz, 1H), 7.01 (d, *J* = 8.3 Hz, 1H), 6.91 - 6.81 (m, 2H), 6.80 - 6.70 (m, 1H), 3.86 (d, J = 1.9 Hz, 3H), 3.82 (d, *J* = 1.9 Hz, 3H), 3.72 (ddd, *J* = 18.3, 12.4, 4.7 Hz, 2H), 3.09 - 2.94 (m, 1H), 2.78 (dd, *J* = 15.4, 3.7 Hz, 1H); ¹³ C NMR (101 MHz, CDCl₃) δ 192.06, 170.08, 168.73, 161.25, 153.55, 149.88, 136.31, 134.57, 132.70, 131.38, 130.40, 126.76, 119.94, 118.65, 118.39, 113.37, 112.85, 112.62, 107.59, 105.96, 102.90, 68.04, 53.97, 52.64, 52.45, 23.14; HRMS (m/z): [M+H]⁺ calcd for C₂₆H₂₃O₇N₂, 475.14998; found, 475.14940.

### Example 26

Characterization of Dimethyl-3-(2-hydroxybenzoyl)-9-methyl-6,7,12,12b-tetrahydro-indolo[2,3-*a*]quinolizine-1,12b-dicarboxylate (1-26):

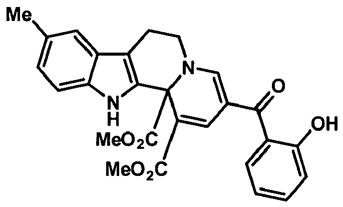

(GP 1) 74%yield; Yellow solid; m.p.: 175 °C; TLC (CH/EA, 3:2 v/v): *R_{F}* = 0,59; ¹H NMR (400 MHz, CDCl₃) δ 11.14 (brs, 1H), 8.94 (s, 1H), 7.95 (d, *J* = 1.5 Hz, 1H), 7.63 (d, *J =* 1.5 Hz, 1H), 7.45 (dd, *J* = 7.8, 1.6 Hz, 1H), 7.41 (ddd, *J =* 8.7, 7.4, 1.7 Hz, 1H), 7.28 - 7.22 (m, 2H), 7.06 - 6.99 (m, 2H), 6.90 - 6.84 (m, 1H), 3.87 (s, 3H), 3.82 (s, 3H), 3.85 - 3.70 (m, 2H), 3.14 (ddd, *J* = 15.5, 11.9, 5.8 Hz, 1H), 2.91 (dd, *J* = 15.4, 3.8 Hz, 1H), 2.44 (s, 3H); ¹³C NMR (101 MHz, CDCl₃) δ 192.01, 170.15, 168.70, 161.35, 153.49, 136.26, 134.51, 131.81, 130.37, 129.20, 126.28, 124.64, 119.96, 118.58, 118.40, 117.94, 113.53, 111.59, 109.91, 107.66, 106.03, 68.08, 53.87, 52.59, 52.55, 23.23, 21.60; HRMS (m/z): [M+H]⁺ calcd for C₂₇H₂₅O₆N₂, 473.17071; found, 473.17013.

### Example 27

Characterization of Dimethyl-9-bromo-3-(2-hydroxybenzoyl)-6,7,12,12b-tetrahydro-indolo[2,3-*a*]quinolizine-1,12b-dicarboxylate (1-27):

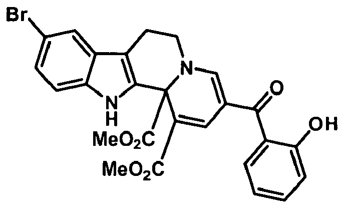

(GP I) 73% yield; yellow solid; m.p.: 182° C; TLC (CH/EA, 3:2 v/v): *R*_{F} = 0.59; ¹H NMR (400 MHz, CDCl₃) δ 11.22 (brs, 1H), 9.17 (s, 1H), 7.96 (d, *J* = 1.5 Hz, 1H), 7.63 - 7.58 (m, 2H), 7.47 - 7.38 (m, 2H), 7.30 - 7.25 (m, 1H), 7.22 (d, J = 8.6 Hz, 1H), 7.06 - 6.98 (m, 1H), 6.94 - 6.81 (m, 1H), 3.87 (s, 3H), 3.83 (s, *J* = 4.4 Hz, 3H), 3.85 - 3.69 (m, 2H), 3.11 (ddd, J = 15.5, 11.8, 5.8 Hz, 1H), 2.87 (dd, J = 15.4, 3.9 Hz, 1H); ¹³C NMR (101 MHz, CDCl₃) δ 192.03, 169.72, 168.78, 161.36, 153.43, 136.57, 134.69, 134.62, 133.11, 130.33, 127.77, 125.83, 121.01, 119.85, 118.62, 118.45, 113.42, 113.26, 113.11, 107.86, 106.12, 67.95, 54.01, 52.69, 52.31, 23.02; HRMS (m/z): [M+H]⁺ calcd for C₂₆H₂₂O₆N₂Br 537.06558; found, 537.06438.

### Example 28

Characterizatiom of (6*S*)-trimethyl 3-(2-hydroxybenzoyl)-6,7,12,12b-tetrahydroindolo[2,3-a]quinolizine-1,6,12b-tricarboxylate

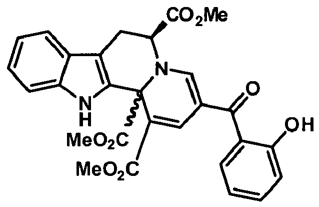

(GP 1) 91 % yield; Amorphous yellow solid; TLC (CH/EA, 3:2 v/v): *R*_{F} = 0,32; Isomer I: ¹H NMR (400 MHz, CDCl₃ ) δ 11.27 (s), 8.92 (s), 7.89 (d, *J* = 1.4 Hz), 7.65 (d, *J* = 1.4 Hz), 7.53 - 7.40 (m), 7.35 (d, *J* = 8.1 Hz), 7.25 - 7.19 (m), 7.15 - 7.09 (m), 7.05 - 6.99 (m), 6.88 (td, *J* = 7.9, 1.2 Hz), 4.58 (t, *J* = 7.7 Hz), 3.91 (s), 3.87 - 3.86 (m, *J* = 2.5 Hz), 3.85 (s), 3.34 - 3.29 (m); ¹³C NMR (101 MHz, CDCl₃) δ 192.62, 170.18, 168.65, 168.26, 161.60, 155.33, 150.75, 136.45, 134.94, 134.70, 131.81, 130.63, 126.00, 123.22, 120.10, 119.61, 118.56, 118.47, 118.32, 115.12, 112.01, 108.43, 108.11, 60.53, 54.17, 53.43, 52.79, 26.75.
Isomer II: ¹H NMR (400 MHz, CDCl₃ ) δ 11.37 (s), 9.32 (s), 7.91 (d, *J* = 1.4 Hz), 7.61 (d, *J* = 1.4 Hz), 7.57 (dd, *J* = 7.9, 1.5 Hz), 7.53 (d, *J* = 7.9 Hz), 7.45 (ddd, *J* = 8.6, 5.8, 1.7 Hz), 7.39 - 7.36 (m), 7.25 - 7.20 (m), 7.12 (ddd, *J =* 8.0, 7.1, 1.0 Hz), 7.03 (dd, *J* = 8.3, 0.9 Hz), 6.94 - 6.89 (m), 4.52 (dd, *J* = 6.3, 1.1 Hz), 3.82 (s, *J* = 5.8 Hz), 3.81 (s), 3.67 (s), 3.56 (dd, J = 15.8, 1.2 Hz), 3.30 (dd, *J* = 15.7, 6.4 Hz); ¹³C NMR (101 MHz, CDCl₃) δ 192.57, 169.30, 168.68, 167.68, 161.61, 155.33, 136.28, 135.52, 134.91, 130.80, 128.38, 125.62, 123.23, 119.97, 119.79, 118.73, 118.49, 118.41, 116.85, 111.90, 108.49, 107.20, 65.55, 53.55, 53.22, 52.51, 23.08; HRMS (m/z): [M+H]⁺ calcd for C₂₈H₂₅O₈N₂, 571.16054; found, 517.15961.

### O-TIPS-Serotonin (A)

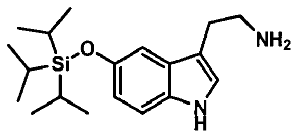

A solution of serotonin hydrochloride (1.10 g; 5.17 mmol), triisopropylchlorosilane (1.33 ml; 6.21 mmol; 1.2 eq.) and imidazole (2.82 g; 41.4 mmol; 8 eq.) in DMF (10 ml) was stirred at room temperature for 5 hours. The mixture was diluted with EA (50 ml) and washed with NaHCO₃ solution (100 ml; 50 ml saturated solution and 50 ml water). The organic layer was dried over MgSO₄ and directly subjected to column chromatography (110g silica gel, EA/MeOH/NH₃ (25% in water) 94:5:1 to 84:15:1). The product was dried under vacuum overnight to yield 1.26 g (3.8 mmol; 73%) of a waxy solid. TLC (EA:MeOH:NH₃(25% in water), 80:20:2 v/v/v): *R*_{F} = 0.36; ¹H NMR (400 MHz, CDCl₃) δ 7.88 (s, 1H), 7.18 (d, *J* = 8.7 Hz, 1H), 7.05 (d, *J* = 2.3 Hz, 1H), 6.99 (s, 1H), 6.80 (dd, *J* = 8.7, 2.3 Hz, 1H), 3.00 (t, *J* = 6.7 Hz, 2H), 2.84 (t, *J* = 6.6 Hz, 2H), 1.51 (s, 4H), 1.35 - 1.21 (m, 3H), 1.12 (d, *J* = 7.3 Hz, 18H); ¹³C NMR (101 MHz, CDCl₃) δ = 149.74, 132.05, 128.35, 122.92, 116.31, 113.66, 111.48, 108.26, 42.53, 29.83, 18.26, 12.95.; HRMS *(m*/*z):* [M+H]⁺ calcd for C₁₉H₃₃ON₂Si, 333.23567; found, 333.23583.

### (B)

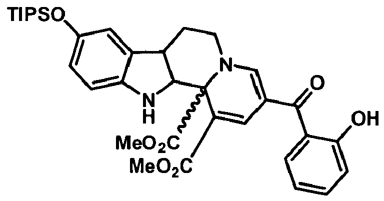

A mixture of 3-formylchromone (252 mg; 1.45 mmol) and toluene (5 ml) was heated to 90°C. To the solution was added DMAD (232 mg; 1.89 mmol; 1.3 eq.) and triphenylphosphine (228 mg; 0.87 mmol; 0.6 eq.). After 5 minutes TLC (DCM/MeOH 100:1) showed complete conversion. Then TIPS-protected serotonin (**A**, 530 mg; 1.6 mmol; 1.1 eq.) was added as a solution in toluene. After 5 minutes TLC (CH/EA 60:40) showed complete conversion. After addition of diphenylphosphate (399 mg; 1.6 mmol; 1.1 eq.) stirring was continued for 15 minutes. TLC (CH/EA 60:40) showed complete conversion. The reaction mixture was directly subjected to column chromatography (silica gel, CH/EA 85:15). Drying under vacuum gave the product (406 mg; 0.64 mmol; 44%) as a yellow amorphous solid. TLC (CH:EA, 3:2v/v): *R*_{F} = 0.64; ¹H NMR (400 MHz, CDCl₃) δ 11.26 (s, 1H), 8.87 (s, 1H), 7.94 (dd, *J* = 5.3, 1.9 Hz, 1H), 7.62 (d, *J* = 1.5 Hz, 1H), 7.45 (dt, *J* = 4.9, 2.4 Hz, 1H), 7.44 - 7.36 (m, 1H), 7.17 (dt, *J* = 8.5, 4.2 Hz, 1H), 7.01 (dt, *J* = 4.4, 2.1 Hz, 1H), 6.90 (d, *J* = 2.3 Hz, 1H), 6.87 (ddd, *J* = 8.4, 6.0, 1.2 Hz, 1H), 6.81 (dd, J = 8.7, 2.3 Hz, 1H), 3.86 (s, 3H), 3.83 (s, 3H), 3.81 - 3.69 (m, 2H), 3.11 (ddd, *J* = 15.4, 12.0, 5.8 Hz, 1H), 2.86 (dd, *J* = 15.4, 3.5 Hz, 1H), 1.34 - 1.21 (m, 3H), 1.11 (d, *J* = 7.0 Hz, 18H); ¹³C NMR (126 MHz, CDCl₃) δ = 191.99, 170.02, 168.67, 161.38, 153.47, 150.02, 136.24, 134.49, 132.33, 131.56, 130.36, 126.72, 119.98, 118.56, 118.41, 117.06, 113.55, 112.22, 107.72, 107.34, 106.04, 68.08, 53.89, 52.56, 23.27, 18.18, 12.87; HRMS (*m*/*z*): [M+H]⁺ calcd for C₃₅H₄₅N₂O₇Si 631.28340; found, 631.28270.

### (C)

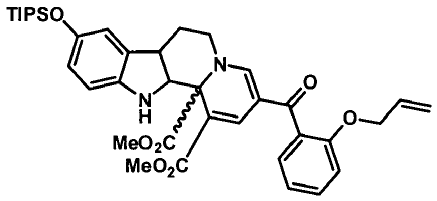

To a suspension of cesium carbonate (530 mg; 1.63 mmol; 3 eq.) in acetone (8 ml) was added TIPS-protected Indoloquinolizine (**B**, 342 mg; 0.542 mmol; 1 eq.) followed by allyl bromide (94 µl; 1.0 mmol; 2 eq.). The mixture was refluxed for two hours, diluted with water and extracted with DCM. The organic layer was dried over MgSO₄ and the solvent was removed. Column chromatography (silica gel, CH/EA 85:15 to 75:25) yielded 346 mg (0.515 mmol; 95%) of the product as a yellow amorphous solid. TLC (CH:EA, 3:2v/v): *R*_{F} = 0.46; ¹H NMR (500 MHz, CDCl₃) δ 8.82 (s, 1H), 7.90 (s, 1H), 7.43 - 7.30 (m, 2H), 7.28 (dd, *J* = 7.5, 1.6 Hz, 1H), 7.14 (d, *J* = 8.7 Hz, 1H), 7.00 (t, *J* = 7.4 Hz, 1H), 6.91 (d, *J* = 8.3 Hz, 1H), 6.86 (d, *J* = 2.2 Hz, 1H), 6.78 (dd, *J* = 8.7, 2.3 Hz, 1H), 5.85 (ddd, *J* = 15.6, 10.4, 5.1 Hz, 1H), 5.25 (dd, *J* = 17.3, 1.1 Hz, 1H), 4.98 (d, *J* = 9.2 Hz, 1H), 4.56 - 4.41 (m, 2H), 3.82 (s, 3H), 3.78 (s, 3H), 3.75 - 3.66 (m, 1H), 3.58 (dd, *J* = 12.9, 5.1 Hz, 1H), 3.03 (ddd, *J* = 17.4, 12.2, 5.6 Hz, 1H), 2.77 (dd, *J* = 15.3, 3.8 Hz, 1H), 1.25 (se, *J* = 7.4 Hz, 3H), 1.10 (d, J = 7.4 Hz, 18H); ¹³C NMR (126 MHz, CDCl₃) δ = 188.65, 170.11, 169.04, 155.51, 153.84, 149.88, 136.15, 132.86, 132.74, 131.52, 131.14, 129.74, 129.46, 126.79, 121.31, 117.31, 116.83, 113.16, 112.15, 107.69, 107.54, 107.29, 69.48, 68.10, 53.73, 52.37, 52.08, 23.33, 18.17, 12.85; HRMS (*m*/*z*): [M+H]⁺ calcd for C₃₈H₄₉N₂O₇Si, 671,31470; found, 671,31401.

### (D)

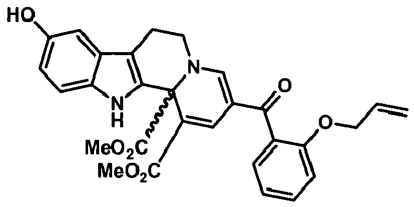

To a solution of fully-protected indoloquinolizine **C** (410 mg; 611 µmol) in THF (10 ml) was added tetrabutylammonium fluoride (208 mg; 794 µmol; 1.3 eq.). The color changed from yellow to brown immediately. After 1 minute TLC (CH/EA 30:70) showed the absence of starting material. The solution was taken up in 20 ml saturated NaHCO₃ solution and extracted with EA. The organic layer was washed with saturated NaHCO₃ solution, saturated NH₄Cl solution, and water. After drying over MgSO₄ the solvent was removed under reduced pressure. To the crude product was added methanol (20 ml) and the product precipitated as yellow solid. The mixture was cooled to 0°C and filtered. The residue was washed with little methanol. Drying under vacuum yielded the product (266 mg; 517 µmol; 85%) as yellow powder. m.p.: 244 °C (decomposition); TLC (EA): *R*_{F} = 0.50; ¹H NMR (500 MHz, DMSO) δ 9.37 (s, 1H), 8.67 (s, 1H), 7.66 (s, 1H), 7.62 (s, 1H), 7.41 (t, *J* = 7.8 Hz, 1H), 7.26 (d, *J* = 8.7 Hz, 1H), 7.18 (d, *J* = 6.2 Hz, 1H), 7.08 (d, *J* = 8.4 Hz, 1H), 7.01 (t, *J* = 7.4 Hz, 1H), 6.72 (d, *J* = 1.8 Hz, 1H), 6.64 (dd, *J* = 8.7, 2.0 Hz, 1H), 5.82 (ddd, *J =* 15.2, 9.4, 4.2 Hz, 1H), 5.21 (d, *J* = 17.2 Hz, 1H), 4.91 (d, *J* = 9.5 Hz, 1H), 4.53 (d, *J* = 4.5 Hz, 2H), 4.13 - 3.99 (m, 1H), 3.81 (s, 3H), 3.71 (s, 3H), 3.43 (dd, *J* = 12.0, 8.2 Hz, 1H), 2.98 - 2.84 (m, 1H), 2.70 (dd, *J* = 15.5, 4.6 Hz, 1H); ¹³C NMR (101 MHz, DMSO) δ = 187.42, 170.03, 166.57, 154.78, 153.82, 150.72, 133.87, 133.07, 132.44, 130.94, 130.65, 128.96, 126.55, 120.79, 116.70, 112.97, 112.76, 112.14, 111.80, 106.29, 105.86, 101.75, 68.31, 67.22, 53.42, 52.30, 49.76, 48.64, 22.46; HRMS (*m*/*z*): [M+H]⁺ calcd for C₂₉H₂₆N₂O₇, 515.18128; found, 515.18027.

### HPLC separation of enantiomers of D

The racemic allyl protected indoloquinolizine was resolved by chiral HPLC using a Dionex UltiMate3000 and a Daicel Chiralpak IC column (10x260 mm) eluting with isohexane/DCM/EtOH (20:80:1.6) at 4 ml/min. The racemic mixture (60 mg) was weighed into a round bottom flask and dissolved in 20 ml CHCl₃/MeOH (9:1) by sonication. The mixture was narrowed to about 1 ml and DCM (6 ml) was added. The mixture was again narrowed to about 1 ml and DCM (3 ml) was added. The sample was filtered and injected to HPLC immediately. The (R)-enantiomer eluted after 10.5 minutes, the (S)-enantiomer after 16 minutes. The pure enantiomers were isolated as amorphous yellow solid.

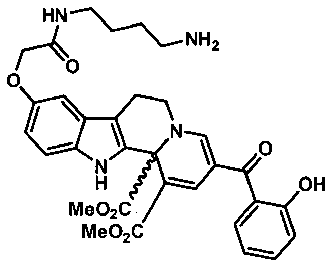

TLC (CHCl₃:MeOH, 7:3v/v): *R*_{F} = 0.38; ¹H NMR (400 MHz, CD₃OD) δ 7.88 (d, *J* = 1.3 Hz, 1H), 7.71 (s, 1H), 7.38 - 7.27 (m, 3H), 6.97 (d, *J* = 2.3 Hz, 1H), 6.89 (dd, *J* = 11.8, 5.3 Hz, 3H), 4.50 (s, 2H), 3.93 - 3.88 (m, 1H), 3.86 (s, 3H), 3.81 - 3.74 (m, 3H), 3.68 (td, *J* = 12.8, 4.6 Hz, 1H), 3.33 - 3.28 (m, 2H), 3.12 - 2.96 (m, 1H), 2.89 - 2.73 (m, 3H), 1.56 (tt, *J* = 10.5, 5.2 Hz, 2H), 1.31 (dd, *J* = 34.0, 18.0 Hz, 2H); ¹³C NMR (101 MHz, CD₃OD) δ = 191.30, 170.71, 170.37, 168.21, 157.58, 154.68, 152.25, 134.84, 132.85, 132.67, 132.38, 129.84, 126.49, 123.68, 119.03, 116.88, 113.38, 112.80, 112.73, 107.70, 106.38, 101.44, 68.06, 67.91, 52.99, 51.73, 51.27, 39.70, 38.15, 26.38, 26.19, 22.68; HRMS (*m*/*z*): [M+H]⁺ calcd for C₃₂H₃₅N₄O₈, 603.24494; found.

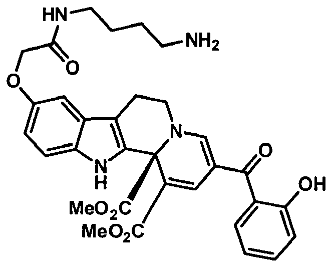

The synthesis was carried out as described for the racemic molecule using the boc-protected *R*-Indoloquinolizine (7.9 mg; 11.2 µmol). [α]^{D}₂₀ (deg cm³ g⁻¹ dm⁻¹) = -103 (*c* = 0.0043 g cm⁻³ in CHCl₃).

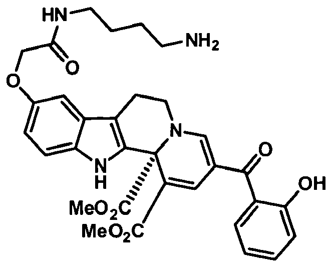

The synthesis was carried out as described for the racemic molecule using the boc-protected S-Indoloquinolizine (7.0 mg; 9.96 µmol). [α]^{D}₂₀ (deg cm³ g⁻¹ dm⁻¹) +95 (c = 0.0043 g cm⁻³ in CHCl₃).

### Example 29

Preparation of 3-(2-Hydroxybenzoyl)-7,12-dihydro-6*H*-indolo[2,3-*a*]quinolizine-1-carboxylic acid methyl ester (**1-29**): The imine (1.2 eq., 0.6 mmol) was dissolved in toluene (5mL) in a Radley Reaction Glass Tube, and the solution was heated to 80°C. After full dissolution of the imine, the chromone-diene (1.0 eq., 0.5 mmol) and ZnCl₂ (20 mol%, 0.1 mmol) were added to the mixture, and was left stirring overnight. When TLC or LCMS showed total disparition of the chromone-diene, the solution was allowed to cool down to room temperature. 25 mL of DCM were added to the solution and washed twice with brine, and the organic layer was dried over Na₂SO₄ and reduced by vacuum. The residue was then purified through preparative HPLC (10 to 100% water in acetonitrile on C18 nucleodur column) with a retention time of 6min.

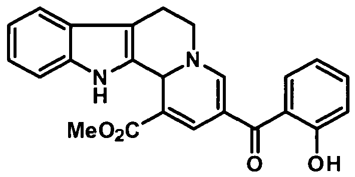

(GP IA) **1-29**: After purification 0.19 mmol (76 mg, 38% yield) of the desired compound was isolated as a yellow solid; Rf = 0.55 (cyclohexane - ethyl acetate 70:30); ¹H NMR (400 MHz, CDCl₃) δ 11.34 (s, 1H), 8.76 (s, 1H), 7.86 (d, J = 1.0 Hz, 1H), 7.68 (d, J = 1.5 Hz, 1H), 7.50 - 7.29 (m, 4H), 7.13 - 7.08 (m, 4H), 6.30 (s, 1H), 3.95 (s, 3H), 3.91 (dd, J = 13.1, 5.7 Hz, 1H), 3.70 (td, J = 13.1, 5.7 Hz, 1H), 3.18 (dd, J = 13.8, 3.8 Hz, 1H), 2.98 (dd, J = 13.8, 3.9 Hz, 1H); ¹³C NMR (101 MHz, cdcl₃) δ 191.6, 167.2, 164.5, 149.8, 136.8, 136.1, 133.5, 131.5, 130.9, 127.4, 121.7, 121.5, 120.9, 120.2, 117.4, 116.5, 116.0, 113.2, 111.1, 108.2, 67.3, 54.1, 50.1, 23.3; HRMS: Calcd for C₂₄H₂₀O₄N₂ [M+H]⁺: 401.14958, Found: 401.14870.

### Example 30

Compound 1-30 was prepared according to the procedure as disclosed for compound 1-29. (1-30):

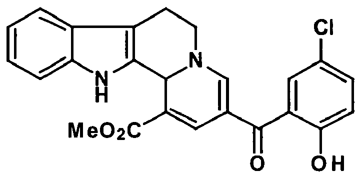

(GP IA) **1-30**: 3-(5-Chloro-2-hydroxybenzoyl)-7,12-dihydro-6H-indolo[2,3-a]quinolizine-1-carboxylic acid methyl ester : After purification 0.22 mmol (95 mg, 45% yield) of the desired compound was isolated as a yellow solid; Rf = 0.45 (cyclohexane - ethyl acetate 70:30); ¹H NMR (400 MHz, CDCl₃) δ 11.55 (s, 1H), 8.74 (s, 1H), 8.08 (s, 1H), 7.88 (d, J = 3.0 Hz, 1H), 7.70 (d, J = 3.5 Hz, 1H), 7.62 (d, J = 6.2 Hz, 1H), 7.13 - 7.09 (m, 4H), 7.01 (d, J = 6.2 Hz, 1H), 6.30 (s, 1H), 3.95 (s, 3H), 3.91 (dd, J = 13.1, 5.7 Hz, 1H), 3.70 (td, J = 13.1, 5.7 Hz, 1H), 3.18 (dd, J = 14.8, 3.8 Hz, 1H), 2.98 (dd, J = 14.8, 3.9 Hz, 1H); ¹³C NMR (101 MHz, cdcl₃) δ 190.4, 168.3, 164.0, 149.1, 137.2, 136.1, 134.9, 131.5, 130.9, 129.4, 127.5, 121.5, 120.9, 120.3, 117.4, 116.9, 116.4, 113.2, 111.3, 108.6, 67.8, 54.3, 50.4, 23.3; LCMS: Calcd for C₂₄H₁₉O₄N₂Cl [M+H]⁺: 434.10, Found: 434.14.

### Example 31

Compound 1-31 was prepared according to the procedure as disclosed for compound 1-29. (1-31):

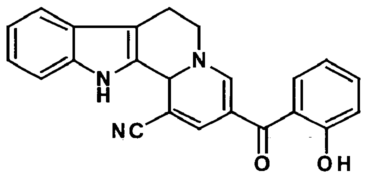

(GP IA) **1-31**: 3-(2-Hydroxybenzoyl)-7,12-dihydro-6*H*-indolo[2,3-*a*]quinolizine-1-carbonitrile: After purification 0.055 mmol (20 mg, 11 % yield) of the desired compound was isolated as a yellow solid; Rf = 0.60 (cyclohexane - ethyl acetate 70:30); ¹H NMR (400 MHz, CDCl₃) δ 11.34 (s, 1H), 8.76 (s, 1H), 7.86 (d, J = 1.0 Hz, 1H), 7.68 (d, J = 1.5 Hz, 1H), 7.34 - 7.26 (m, 4H), 7.13 - 7.09 (m, 4H), 6.29 (s, 1H), 3.91 (dd, J = 13.1, 5.7 Hz, 1H), 3.70 (td, J = 13.1, 5.7 Hz, 1H), 3.18 (dd, J = 14.8, 3.8 Hz, 1H), 2.98 (dd, J = 14.8, 3.9 Hz, 1H); ¹³C NMR (101 MHz, cdcl₃) δ 190.6, 167.4, 153.2, 135.8, 135.5, 134.1, 131.2, 130.7, 125.6, 122.0, 121.7, 120.9, 120.2, 118.0, 115.6, 115.2, 114.6, 113.2, 111.5, 105.7, 60.3, 56.3, 23.5; HRMS: Calcd for C₂₃H₁₇O₂N₃ [M+H]⁺: 368.13935, Found: 368.13917.

### Example 32

Compound 1-32 was prepared according to the procedure as disclosed for compound 1-29. (1-32):

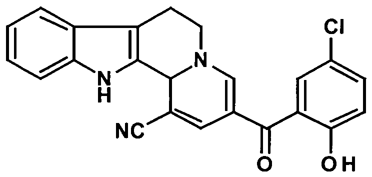

(GP IA) **1-32**: 3-(5-Chloro-2-hydroxybenzoyl)-7,12-dihydro-6*H*-indolo[2,3-a]quinolizine-1-carbonitrile: After purification 0.075 mmol (30 mg, 15% yield) of the desired compound was isolated as a yellow solid; Rf = 0.55 (cyclohexane - ethyl acetate 70:30. ¹H NMR (400 MHz, CDCl₃) δ 11.36 (s, 1H), 8.76 (s, 1H), 8.11 (s, 1H), 7.84 (d, J = 1.0 Hz, 1H), 7.72 (d, J = 1.5 Hz, 1H), 7.67 (d, J = 5.8 Hz, 1H), 7.12 - 7.08 (m, 4H), 7.00 (d, J = 5.8 Hz, 12H), 6.28 (s, 1H), 3.94 (s, 3H), 3.91 (dd, J = 12.9, 6.0 Hz, 1H), 3.68 (td, J = 12.9, 6.0 Hz, 1H), 3.17 (dd, J = 14.8, 3.6Hz, 1H), 2.99 (dd, J = 14.8, 3.6 Hz, 1H); LCMS: Calcd for C₂₃H₁₆O₄N₂ [M+H]⁺: 402.10, Found: 402.28.

### Example 33

The imine (1.2 eq., 0.30 mmol) was dissolved in ethyl acetate (5mL) in a Radley Reaction Glass Tube, and the solution was heated to 80°C. After full dissolution of the imine, the chromone-diene (1.0 eq., 0.25 mmol) and ZnCl₂ (20 mol%, 0.05mmol) were added to the mixture, that was left stirring for 4 to 48h. When TLC or LCMS showed total disparition of the chromone-diene, the solution was allowed to cool down to room temperature. 25 mL of DCM were added to the solution and washed twice with brine, and the organic layer was dried over Na₂SO₄ and reduced by vacuum. The residue was then purified through MPLC, with a 0% to 100% gradient of ethyl acetate in cyclohexane over 15 minutes on a 12g pre-packed 30µm Puriflash-Interchim silica cartridge. (Compound peak around 50% EtOAc/Cyclohexane)

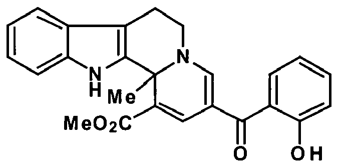

(GP IA) **1-33:** 3-(2-Hydroxybenzoyl)-12b-methyl-7,12-dihydro-6*H*-indolo[2,3-a]-quinolizine-1-carboxylic acid methyl ester (1-33): After purification 0.19 mmol (79 mg, 38% yield) of the desired compound was isolated as a yellow solid; Rf = 0.55 (cyclohexane - ethyl acetate 70:30); ¹H NMR (400 MHz, CDCl₃) δ 11.35 (s, 1H), 8.75 (s, 1H), 7.87 (d, J = 1.0 Hz, 1H), 7.68 (d, J = 1.5 Hz, 1H), 7.50 - 7.29 (m, 4H), 7.13-7.09 (m, 4H), 3.94 (s, 3H), 3.88 (dd, J = 12.1, 5.5 Hz, 1H), 3.70 (td, J = 12.1, 5.5 Hz, 1H), 3.54 (s, 3H), 3.17 (dd, J = 14.8, 3.8 Hz, 1H), 2.99 (dd, J = 14.8, 3.9 Hz, 1H); ¹³C NMR (101 MHz, cdcl₃) δ 193.6, 166.5, 164.6, 149.3, 136.3, 136.1, 133.5, 131.3, 131.3, 127.3, 122.1, 121.2, 120.7, 119.7, 117.1, 116.5, 116.4, 113.2, 110.7, 108.4, 73,4, 59.7, 52.3, 48.1, 19.8. LCMS: Calcd for C₂₅H₂₂O₄N₂ [M+H]⁺: 415.16, Found: 415.12.

### Example 34

Compound 1-34 was prepared according to the procedure as disclosed for compound 1-33.

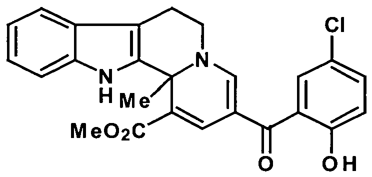

(GP IA) **1-34**: 3-(5-Chloro-2-hydroxybenzoyl)-12b-methyl-7,12-dihydro-6*H*-indolo[2,3-a]quinolizine-1-carboxylic acid methyl ester (1-34): After purification 0.11 mmol (49 mg, 22% yield) of the desired compound was isolated as a yellow solid; Rf = 0.60 (cyclohexane - ethyl acetate 70:30); ¹H NMR (400 MHz, CDCl₃) δ 11.31 (s, 1H), 8.74 (s, 1H), 8.09 (s, 1H), 7.83 (d, J = 3.0 Hz, 1H), 7.74 (d, J = 3.5 Hz, 1H), 7.67 (d, J = 6.0 Hz, 1H), 7.12 - 7.08 (m, 4H), 7.01 (d, J = 6,1 Hz), 3.97 (s, 3H), 3.91 (dd, J = 13.1, 5.7 Hz, 1H), 3.70 (td, J = 13.1, 5.7 Hz, 1H), 3.51 (s, 3H), 3.18 (dd, J = 15.0, 4.0 Hz, 1H), 2.98 (dd, J = 15.0, 4.0 Hz, 1H); LCMS: Calcd for C₂₅H₂₁O₄N₂Cl [M+H]⁺: 449.13, Found: 449.62.

### Example 35

Compound 1-35 was prepared according to the procedure as disclosed for compound 1-33. 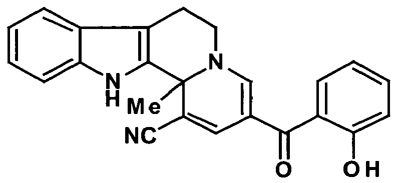 (GP IA) **1-35:** 3-(2-Hydroxybenzoyl)-12b-methyl-7,12-dihydro-6*H*-indolo[2,3-a]-quinolizine-1-carbonitrile (1-40): After purification 0.17 mmol (65 mg, 34% yield) of the desired compound was isolated as a yellow solid; Rf = 0.55 (cyclohexane - ethyl acetate 70:30); ¹H NMR (400 MHz, CDCl₃) δ 11.36 (s, 1H), 8.76 (s, 1H), 7.95 (d, J = 1.0 Hz, 1H), 7.72 (d, J = 1.5 Hz, 1H), 7.45 - 7.29 (m, 4H), 7.14-7.10 (m, 4H), 3.83 (dd, J = 12.8, 5.1 Hz, 1H), 3.70 (td, J = 12.8, 5.1 Hz, 1H), 3.88 (s, 3H), 3.17 (dd, J = 13.9, 3.8 Hz, 1H), 2.99 (dd, J = 13.9, 3.9 Hz, 1H); LCMS: Calcd for C₂₄H₁₉O₂N₃ [M+H]⁺: 382.16, Found: 382.20.

### Example 36

Compound 1-36 was prepared according to the procedure as disclosed for compound 1-33.

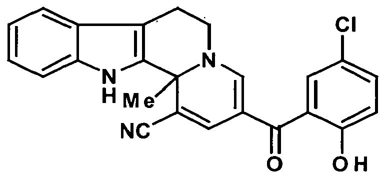

(GP IA) **1-36:** 3-(5-Chloro-2-hydroxybenzoyl)-12b-methyl-7,12-dihydro-6H-indolo[2,3-a]quinolizine-1-carbonitrile (1-36): After purification 0.13 mmol (54 mg, 26% yield) of the desired compound was isolated as a yellow solid; Rf = 0.55 (cyclohexane - ethyl acetate 70:30¹H NMR (400 MHz, CDCl₃) δ 11.33 (s, 1H), 8.76 (s, 1H), 8.09 (s, 1H), 7.78 (d, J = 3.0 Hz, 1H), 7.70 (d, J = 5.5 Hz, 1H), 7.66 (d, J = 3.3 Hz, 1H), 7.13 - 7.08 (m, 4H), 7.03 (d, J = 5.5 Hz, 1H), 3.91 (dd, J = 13.1, 5.7 Hz, 1H), 3.76 (td, J = 13.1, 5.7 Hz, 1H), 3.49 (s, 3H), 3.16 (dd, J = 15.0, 4.0 Hz, 1H), 2.96 (dd, J = 15.0, 4.0 Hz, 1H); LCMS: Calcd for C₂₄H₁₈O₄N₂Cl [M+H]⁺: 416.12, Found: 416.24.

### Example 37

Compound 1-37 was prepared according to the procedure as disclosed for compound 1-33.

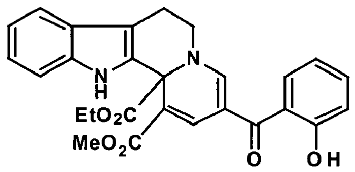

(GP IA) **1-37**: Characterization of 12b-ethyl 1-methyl 3-(2-hydroxybenzoyl)-6,7,12,12b-tetrahydroindolo[2,3-a]quinolizine-1,12b-dicarboxylate (1-37): After purification 0.01 mmol (4 mg, 2% yield) of the desired compound was isolated as a yellow solid; Rf = 0.50 (cyclohexane - ethyl acetate 60:40); LCMS: Calcd for C₂₇H₂₄O₆N₂ [M+H]⁺: 473.17, Found: 473.44.

### Example 38

Compound 1-38 was prepared according to the procedure as disclosed for compound 1-33.

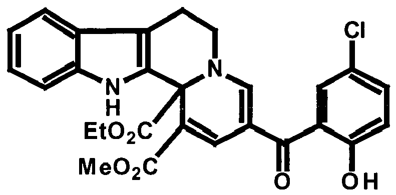

(GP IA) **1-38:** Characterization of 12b-ethyl 1-methyl 3-(5-chloro-2-hydroxybenzoyl)-6,7,12,12b-tetrahydroindolo[2,3-a]quinolizine-1,12b-dicarboxylate (1-38): After purification 0.01 mmol (4 mg, 2% yield) of the desired compound was isolated as a yellow solid; Rf = 0.45 (cyclohexane - ethyl acetate 60:40); LCMS: Calcd for C₂₇H₂₃O₆N₂Cl [M+H]⁺: 507.13, Found: 507.24.

### Example 39

Compound 1-39 was prepared according to the procedure as disclosed for compound 1-33.

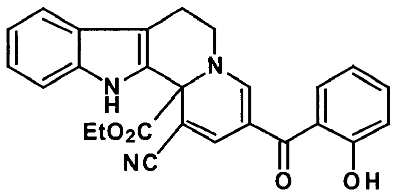

(GP IA) **1-39:** ethyl 1-cyano-3-(2-hydroxybenzoyl)-6,7,12,12b-tetrahydroindolo[2,3-a]quinolizine-12b-carboxylate (1-39): Rf = 0.5 (cyclohexane - ethyl acetate 60:40), yield 10%

### Example 40

Compound 1-40 was prepared according to the procedure as disclosed for compound 1-33.

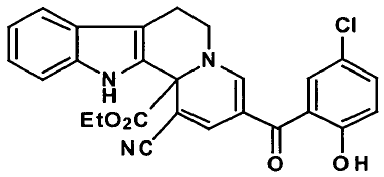

(GP IA) **1-40**: ethyl 3-(5-chloro-2-hydroxybenzoyl)-1-cyano-6,7,12,12b-tetrahydro-indolo[2,3-a]quinolizine-12b-carboxylate (1-40): Rf = 0.5 (cyclohexane - ethyl acetate 60:40), yield 10%.

### Example 41

Preparation of 3-(2-Hydroxybenzoyl)-7,12-dihydro-6*H*-indolo[2,3-a]quinolizine-1-carboxylic acid methyl ester (1-29): The imine (1.2 eq., 0.6 mmol) was dissolved in toluene (5mL) in a Radley Reaction Glass Tube, and the solution was heated to 80°C. After full dissolution of the imine, the chromone-diene (1.0 eq., 0.5 mmol) and ZnCl₂ (20 mol%, 0.1 mmol) were added to the mixture, and was left stirring overnight. When TLC or LCMS showed total disparition of the chromone-diene, the solution was allowed to cool down to room temperature. 25 mL of DCM were added to the solution and washed twice with brine, and the organic layer was dried over Na₂SO₄ and reduced by vacuum. The residue was then purified through preparative HPLC (10 to 100% water in acetonitrile on C18 nucleodur column) with a retention time of 6min.

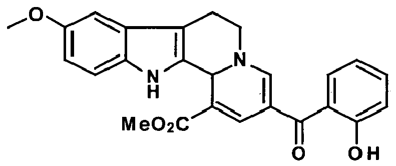

**1-41:** Characterization of 3-(2-Hydroxybenzoyl)-9-methoxy-7,12-dihydro-6*H*-indolo[2,3-a]quinolizine-1-carboxylic acid methyl ester (1-41): After purification 0.40 mmol (172 mg, 80% yield) of the desired compound was isolated as a yellow solid; Rf = 0.45 (cyclohexane - ethyl acetate 70:30); ¹H NMR (400 MHz, CDCl₃) δ 11.29 (s, 1H), 8.70 (s, 1H), 7.82 (d, J = 1.0 Hz, 1H), 7.71 (d, J = 1.2 Hz, 1H), 7.50 - 7.29 (m, 4H), 7.24 - 7.19 (m, 3H), 6.30 (s, 1H), 4.10 (s, 3H), 3.95 (s,3H) 3.91 (dd, J = 12.9, 5.7 Hz, 1H), 3.70 (td, J = 12.9, 5.7 Hz, 1H), 3.18 (dd, J = 14.8, 3.6 Hz, 1H), 2.98 (dd, J = 14.5, 3.9 Hz, 1H); ¹³C NMR (101 MHz, cdcl₃) δ 188.9, 167.4, 164.1, 150.4, 137.8, 136.7, 133.4, 131.5, 131.1, 127.7, 121.8, 121.0, 120.7, 120.0, 119.2, 117.4, 116.5, 116.0, 113.2, 108.2, 67.3, 56.1, 54.2, 48.3, 23.8; HRMS: Calcd for C₂₅H₂₂O₅N₂ [M+H]⁺: 431.16015, Found: 431.15826.

### Example 42

Compound 1-42 was prepared according to the procedure as disclosed for compound 1-41.

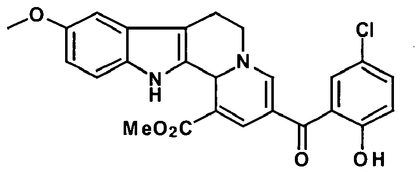

**1-42:** 3-(5-Chloro-2-hydroxybenzoyl)-9-methoxy-7,12-dihydro-6*H*-indolo[2,3-a]-quinolizine-dicarboxylic acid 1-methyl-12b-ethyl ester (1-42): Rf = 0.4 (cyclohexane-ethyl acetate 70:30), yield 10%.

### Example 43

Compound 1-43 was prepared according to the procedure as disclosed for compound 1-41.

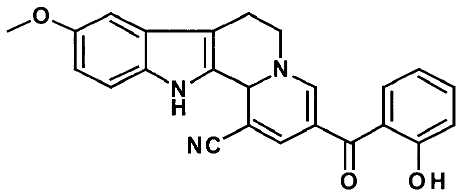

**1-43:** Characterization of 3-(2-Hydroxybenzoyl)-9-methoxy-7,12-dihydro-6*H*-indolo[2,3-a]quinolizine-1-carbonitrile (1-43): After purification 0.015 mmol (6 mg, 3% yield) of the desired compound was isolated as a yellow solid; Rf = 0.55 (cyclohexane - ethyl acetate 70:30), LCMS: Calcd for C₂₄H₁₉O₃N₃ [M+H]⁺: 398.15, Found: 398.33

### Example 44

Compound 1-44 was prepared according to the procedure as disclosed for compound 1-41.

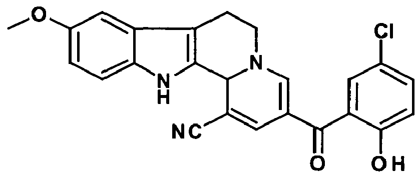

1-44: 3-(5-Chloro-2-hydroxybenzoyl)-9-methoxy-7,12-dihydro-6*H*-indolo[2,3-a]-quinolizine-1-carbonitrile (1-44): Rf = 0.55 (cyclohexane - ethyl acetate 70:30), yield 10%.

### Example 45

The imine (1.2 eq., 0.30 mmol) was dissolved in DCM (5mL) in a Radley Reaction Glass Tube, and the solution was stirred at room temperature under argon atmosphere. After full dissolution of the imine, the chromone-diene (1.0 eq., 0.25 mmol) and ZnEt₂ (20 mol%, 0.05mmol) were added to the mixture, that was left stirring for 5min to 8h. When TLC or LCMS showed total disparition of the chromone-diene, the solution was allowed to cool down to room temperature. 25 mL of DCM were added to the solution and washed twice with brine, and the organic layer was dried over Na₂SO₄ and reduced by vacuum. The residue was then purified through MPLC, with a 0% to 100% gradient of ethyl acetate in cyclohexane over 15 minutes on a 12g pre-packed 30µm Puriflash-Interchim silica cartridge. (Compound peak around 50% EtOAc/Cyclohexane)

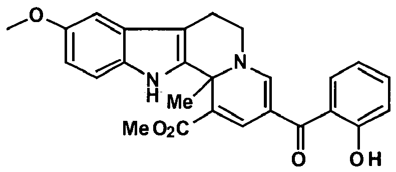

**1-45:** 3-(2-Hydroxybenzoyl)-9-methoxy-12b-methyl-7,12-dihydro-6*H*-indolo[2,3-a]quinolizine-1-carboxylic acid methyl ester (1-45): After purification 0.45 mmol (200 mg, 90% yield) of the desired compound was isolated as a yellow solid; Rf = 0.55 (cyclohexane - ethyl acetate 70:30); ); ¹H NMR (400 MHz, CDCl₃) δ 11.31 (s, 1H), 8.72 (s, 1H), 7.80 (d, J = 1.0 Hz, 1H), 7.74 (d, J = 1.2 Hz, 1H), 7.47 - 7.30 (m, 4H), 7.23 - 7.18 (m, 3H), 4.11 (s, 3H), 3.97 (s,3H), 3.91 (dd, J = 12.3, 6.3 Hz, 1H), 3.68 (td, J = 12.4, 6.3 Hz, 1H), 3.53 (s, 3H), 3.16 (dd, J = 13.8, 3.2 Hz, 1H), 3.02 (dd, J = 13.8, 3.2 Hz, 1H); ¹³C NMR (101 MHz, cdcl₃) δ 191.6, 164.2, 164.5, 150.8, 135.8, 135.1, 132.5, 131.5, 131.7, 127.7, 123.4, 121.5, 120.4, 119.6, 117.4, 116.5, 116.0, 113.2, 111.7, 104.2, 70.2, 67.7, 55.4, 52.9, 49.8, 24.3.; HRMS: Calcd for C₂₆H₂₄O₄N₂ [M+H]⁺: 445.17580, Found: 445.17561.

### Example 46

Compound 1-46 was prepared according to the procedure as disclosed for compound 1-45.

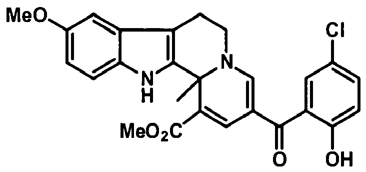

**1-46**: 3-(5-Chloro-2-hydroxybenzoyl)-9-methoxy-12b-methyl-7,12-dihydro-6*H-*indolo[2,3-a]quinolizine-l-carboxylic acid methyl ester (1-46): After purification 0.46 mmol (220 mg, 92% yield) of the desired compound was isolated as a yellow solid; Rf = 0.55 (cyclohexane - ethyl acetate 70:30); ¹H NMR (400 MHz, CDCl₃) δ 11.52 (s, 1H), 8.76 (s, 1H), 8.12 (s, 1H), 7.77 (d, J = 3.0 Hz, 1H), 7.70 (d, J = 3.5 Hz, 1H), 7.56 (d, J = 5.8 Hz, 1H), 7.14 - 7.10 (m, 4H), 7.05 (d, J = 5.8 Hz, 1H), 4.12 (s, 3H), 3.95 (s, 3H), 3.94 (dd, J = 14.0, 5.2 Hz, 1H), 3.88 (s, 3H), 3.70 (td, J = 14.0, 5.2 Hz, 1H), 3.18 (dd, J = 14.8, 3.8 Hz, 1H), 2.98 (dd, J = 14.8, 3.9 Hz, 1H); HRMS: Calcd for C₂₆H₂₄O₅N₂Cl [M+H]⁺: 478.13683, Found: 479.13625.

### Example 47

Compound 1-47 was prepared according to the procedure as disclosed for compound 1-45.

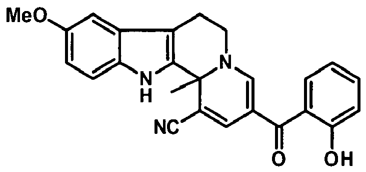

**1-47:** 3-(2-Hydroxybenzoyl)-9-methoxy-12b-methyl-7,12-dihydro-6*H*-indolo[2,3-a]quinolizine-1-carbonitrile (1-47): Yield : 80%, Rf = 0.5 (Cyclohexane - ethyl acetate 70 : 30)

### Example 48

Compound 1-48 was prepared according to the procedure as disclosed for compound 1-45.

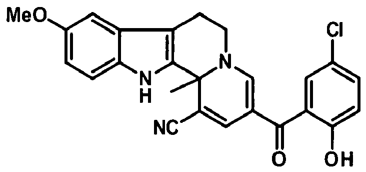

**1-48:** 3-(5-Chloro-2-hydroxybenzoyl)-9-methoxy-12b-methyl-7,12-dihydro-6*H-*indolo[2,3-a]quinolizine-1-carbonitrile (1-48): After purification 0.42 mmol (187 mg, 84% yield) of the desired compound was isolated as a yellow solid; Rf = 0.55 (cyclohexane - ethyl acetate 70:30); ¹H NMR (400 MHz, CDCl₃) δ 11.49 (s, 1H), 8.8 6 (s, 1H), 8.05 (s, 1H), 7.89 (d, J = 3.4 Hz, 1H), 7.68 (d, J = 3.5 Hz, 1H), 7.57 (d, J=6.0 Hz, 1H), 7.17 - 7.14 (m, 4H), 7.03 (d, J = 6.0 Hz, 1H), 6.30 (s, 1H), 4.12 (s, 3H), 3.92 (dd, J = 12.5, 5.5 Hz, 1H), 3.86 (s, 3H), 3.66 (td, J = 12.5, 5.5 Hz, 1H), 3.18 (dd, J = 12.8, 4.3 Hz, 1H), 2.98 (dd, J = 12.8, 4.3 Hz, 1H); HRMS: Calcd for C₂₅H₂₁O₃N₃Cl [M+H]⁺: 446.12660, Found: 446.12626.

### Example 49

Preparation of 3-(2-Hydroxybenzoyl)-7,12-dihydro-6*H*-indolo[2,3-a]quinolizine (1-49): The imine (1.2 eq., 0.30 mmol) was dissolved in 1 mL DMSO and 5mL toluene in a Radley Reaction Glass Tube, and the solution was heated to 80°C. After full dissolution of the imine, the chromone-diene (1.0 eq., 0.25 mmol) and ZnCl₂ (20 mol%, 0.05mmol) were added to the mixture, that was left stirring for 48h. When TLC or LCMS showed total disparition of the chromone-diene, the solution was allowed to cool down to room temperature. 25 mL of DCM were added to the solution and washed twice with brine, and the organic layer was dried over Na₂SO₄ and reduced by vacuum. The residue was then purified through MPLC, with a 0% to 100% gradient of ethyl acetate in cyclohexane over 15 minutes on a 12g pre-packed 30µm Puriflash-Interchim silica cartridge. (Compound peak around 35% EtOAc/Cyclohexane) 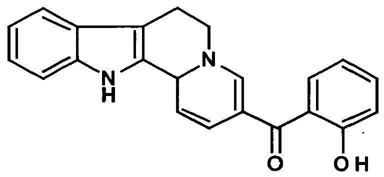 **1-49:** 3-(2-Hydroxybenzoyl)-7,12-dihydro-6*H*-indolo[2,3-a]quinolizine (1-49): Rf = 0.7 (Cyclohexane/EtOAc 70/30); yield 10%.

### Example 50

Compound 1-50 was prepared according to the procedure as disclosed for compound 1-49.

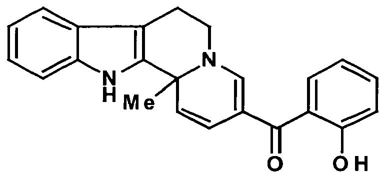

**1-50:** 3-(2-Hydroxybenzoyl)-12b-methyl-7,12-dihydro-6*H*-indolo[2,3-a]quinolizine (1-50): Rf = 0.7 (Cyclohexane/EtOAc 70/30); yield 10%.

### Example 51

Compound 1-51 was prepared according to the procedure as disclosed for compound 1-49.

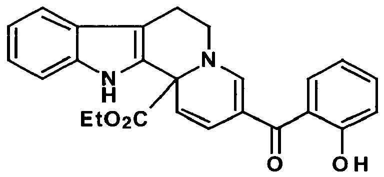

1-51: 3-(2-Hydroxybenzoyl)-7,12-dihydro-6*H*-indolo[2,3-*a*]quinolizine-12b-carboxylic acid ethyl ester (1-51): Rf = 0.7 (Cyclohexane/EtOAc 70/30); yield 10%.

### Example 52

Compound 1-52 was prepared according to the procedure as disclosed for compound 1-49.

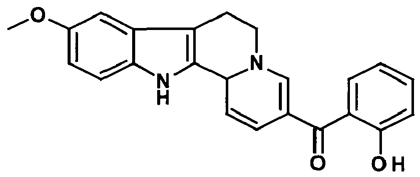

1-52: 3-(2-Hydroxybenzoyl)-9-methoxy-7,12-dihydro-6*H*-indolo[2,3-*a*]quinolizine (1-52): Rf = 0.7 (Cyclohexane/EtOAc 70/30); yield 10%.

### Example 53

Compound 1-53 was prepared according to the procedure as disclosed for compound 1-49.

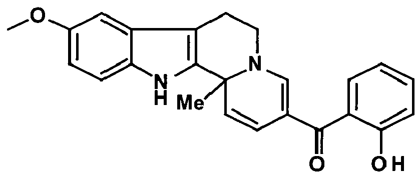

**1-53**: 3-(2-Hydroxybenzoyl)-9-methoxy-12b-methyl-7,12-dihydro-6*H*-indolo[2,3-*a*]quinolizine (1-53): Rf = 0.7 (Cyclohexane/EtOAc 70/30); yield 10%.

### Example 54 and 55: Biological Evaluation of the Indoloquinolizines

### Example 54A und 54B: Effect of Indoloquinolizines (of general formula 1) on Mitotic Cell Division and Cell Cycle

### General Cell culture

HeLa human cervical carcinoma cell line, the human breast cancer cell line MCF7, and the human colorectal carcinoma cell line SW480 were maintained in Dulbecco's Modified Eagle's medium (DMEM, PAA, Pasching Austria). The colorectal cell line HT29 was cultured in McCoy's 5A medium (PAA, Pasching, Austria). DMEM medium was supplemented with 10 % fetal calf serum, sodium pyruvate, non-essential amino acids, penicillin and streptomycin, McCoy's 5A medium was supplemented with 10% fetal calf serum, penicillin and streptomycin. All cell lines were cultured at 37°C in a 5% CO₂ humidified atmosphere.

### Example 54A: Facs analyses of the cell cycle

### Facs analyses

Further, fluorescent activated cell sorting (FACS) was used successfully to analyze the cell cycle and mitotic arrest under the influence of indoloquinolizines.

HeLa cells were fixed and stained for FACS analysis 24h after compound treatment. Cells were trypsinized and combined with any floating cells, pelleted, and resuspended in 1 ml PBS. Ice cold ethanol (70%) was added, and samples were incubated at -20 °C until analysis. After fixation the cells were pelleted, resuspended in 500 µl staining solution (50 µg/ml propidium iodide, 0.1 mg/ml RNase A, 0.05% Triton X-100) and incubated for 40 min at 37 °C. 3 ml PBS were added; the cells were pelleted again and resuspended in 500 µl PBS. The DNA content was measured with a Becton Dickinson LSRII and analyzed with FlowJo 7.5 software.

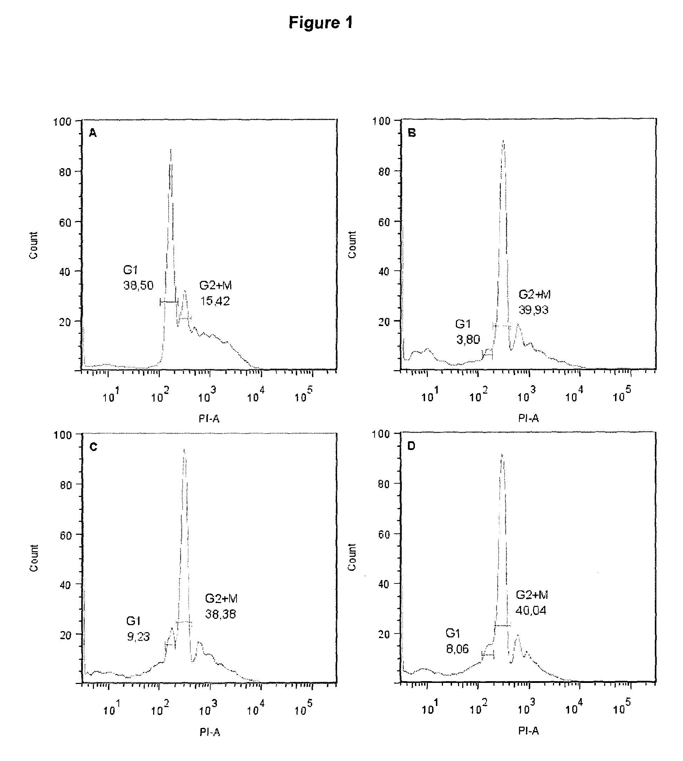

The cell cycle of compound treated HeLa cells was analyzed by fluorescence activated cell sorting (Figure 1). The cell cycle analysis of DMSO treated cells (Figure 1a) shows that 39% of the cells are in G1 phase and 16% are in G2/M phase, whereas in nocodazole treated cells (Figure 1b) only 4% are in G1 phase and 40% are in G2/M phase. A similar distribution is given in (-)-*1-01* treated cells (Figures 1c and 1d); here 9% are in G1 and 38% in G2/M phase (25 µM) respectively; 8% in G1 and 40% in G2/M phase (50 µM). This means that the mitotic index is increased by more than two fold (from 0.16 to 0.38 respectively 0.40), indicating a mitotic arrest after compound treatment.

### Example 54B: Cell cycle analyses by live-Cell Imaging and Immunofluorescence

The phenotypic effects on mitotic divisions of HeLa cells were visualized using immunofluorescence technique.

### Immunofluorescence

Cells grown on coverglasses were permeabilized and fixed by incubating in fixation buffer (3.7 % Formaldehyde, 100 mM K-Pipes pH 6.8, 10 mM EGTA, 1mM MgCl₂, 0.1% Triton X-100) for 20 min. cells were blocked for at least one hour in TBST containing 2% BSA at room temperature. α-tubulin was visualized using rat antibody (AbD serotec) diluted 1:2000 in TBST, 2% BSA. Secondary antibody was developed in rabbit and coupled to Alexa488 (Invitrogen). DNA was visualized by staining with Hoechst33342 (Invitrogen). Cells were mounted with Aqua-Poly/Mount mounting medium (Polysciences). Fluorescence images were captured by using a Leica TCS SP5 laser confocal microscope.

### Immunofluorescence

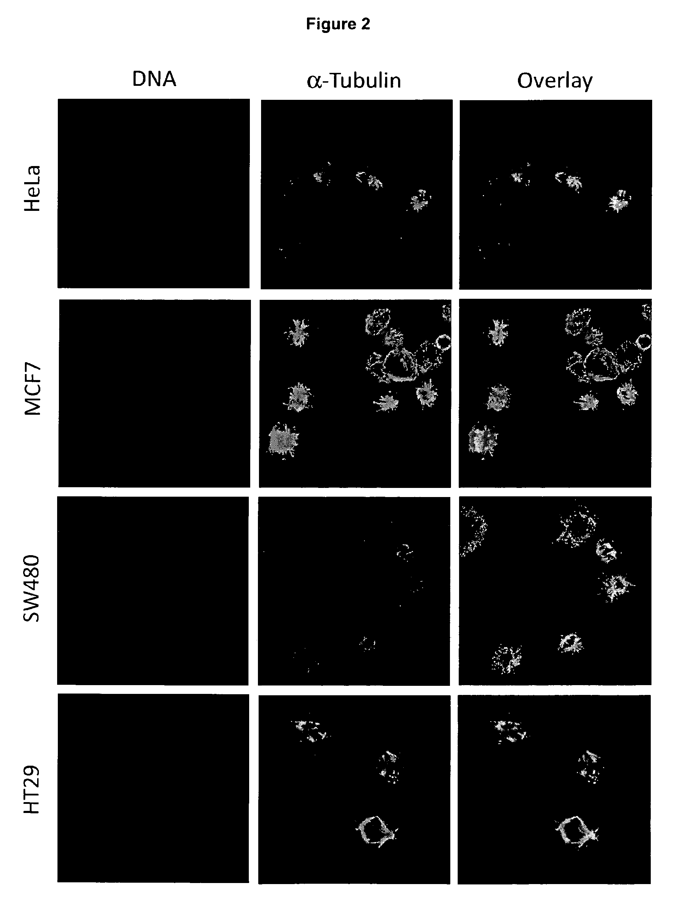

Immunofluorescence images of HeLa, MCF7, SW480 and HT29 cells stained for α-tubulin and DNA (Figure 2) revealed a clear misalignment of the chromosomes during mitosis. This phenotype could already be observed at low µM (1 µM) concentrations. Additionally in some mitotic cells, treated with compound concentrations of 25 µM and higher, a tripolarization could be observed.

### Example 55A and 55B: Effect of Indoloquinolizines of general formula 1 on Cell viability and proliferation and cell apoptosis.

### General cell culture

HeLa human cervical carcinoma cell line, the human breast cancer cell line MCF7, the African green monkey kidney cell line BS-C-1 and the human colorectal carcinoma cell lines HCT116 and SW480 were maintained in Dulbecco's Modified Eagle's medium (DMEM, PAA, Pasching Austria). The colorectal cell line HT29 was cultured in McCoy's 5A medium (PAA, Pasching, Austria). The prostate carcinoma cells PC3 and the osteosarcoma cells Saos2 were cultured in RPMI medium (PAA, Pasching Austria). All media were supplemented with 10 % fetal calf serum, L-glutamine, penicillin and streptomycin. All cell lines were maintained at 37°C in a 5% CO₂ humidified atmosphere.

### Example 55A: Cell viability and proliferation assay

The cell proliferation was determined as a function of the metabolic activity by means of the cell proliferation reagent WST-1 (Roche, Mannheim Germany). Cells were plated in quadruplicates into the wells of 96 well plates and were allowed to attach for 24 hours. Cells were then treated with different concentrations of the compounds for 19 hours. All samples contained 0.5% DMSO. Control cells were treated with 0.5% DMSO or 20 µM doxorubicin or 1 µM staurosporine. After the treatment the WST-1 reagent was added to the cells according to the manufacturer's protocol. The absorbance was measured with the TECAN Inifinite^{®} M200 plate reader at 450/690 nm. The blank value was subtracted from each value and the percentage of viable cells was determined compared to the DMSO treated cells.

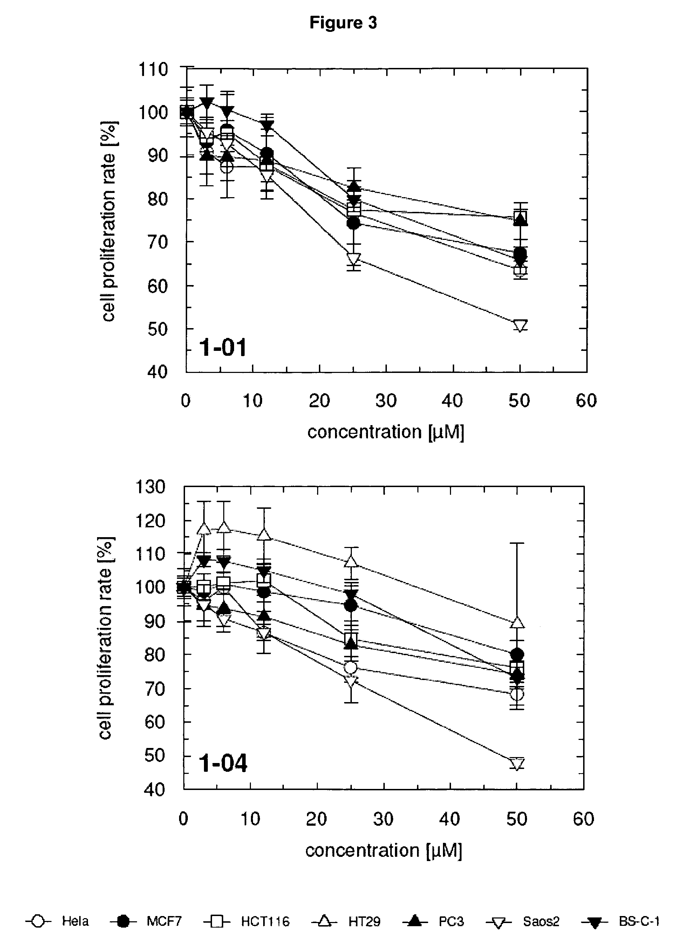

The biological activity of the indoloquinolizines was evaluated in different cell lines by means of WST-1 assay. Cells were treated with different compound concentrations (3.125 up to 50 µM) for 19 h. In HeLa cells the (-)-enantiomers of **1-01** and **1-04** caused the most dramatic decrease in cell proliferation rate with increasing concentrations (Figure 3). At 50 µM concentration (-)-**1-01** reduced the cell proliferation rate to 60 % and (-)-**1-04** to 67 % (Figure 3). The calculated IC₅₀ values in HeLa cells for (-)-**1-01** and (-)-**1-04** are 28.43 ± 0.61µM and 15.70 ± 1.43 µM respectively (calculated from two and three experiments respectively). Similar results were obtained for the breast cancer cell line MCF7, the colorectal cell line HCT116 and for the African green monkey kidney cell BS-C-1. The decrease in the proliferation rate was more dramatic in the osteosarcoma cells Saos2, whereas we detected only a slight reduction in cell viability in the prostate carcinoma cells PC3 and in the colorectal cell line HT29.

### Example 55B: Cell apoptosis assay

Since the most active compounds of the present invention have strong antiproliferative effect in the many of the tested cell lines and caused prolonged mitosis in HeLa cells, we addressed the question whether this is followed by apoptosis induction. Therefore we measured the caspase-3/7 activity in different cell lines with the Apo-ONE^{®} homogeneous caspase-3/7 assay.

Caspase-3/7 activities were determined using the Apo-ONE^{®} homogeneous caspase-3/7 assay (Promega, Mannheim Germany) according to the manufacturer's protocol. Briefly, cells were plated in quadruplicates in 384-well cell culture plate. After 24 hours cells were exposed for 42 hours to the compounds at different concentrations. Control cells were treated with 0.5 % DMSO or staurosporine (STS). Subsequently cells were lysed with a lysis buffer containing the caspase-3/7 substrate Z-DEVD-R110 and were incubated at room temperature until further analysis. The fluorescence was detected with the TECAN Inifinite^{®} M200 plate reader at excitation/emission wavelength 485/520 nm. The blank value was subtracted from each value and the relative caspase-3/7 activity was calculated compared to the DMSO treated cells.

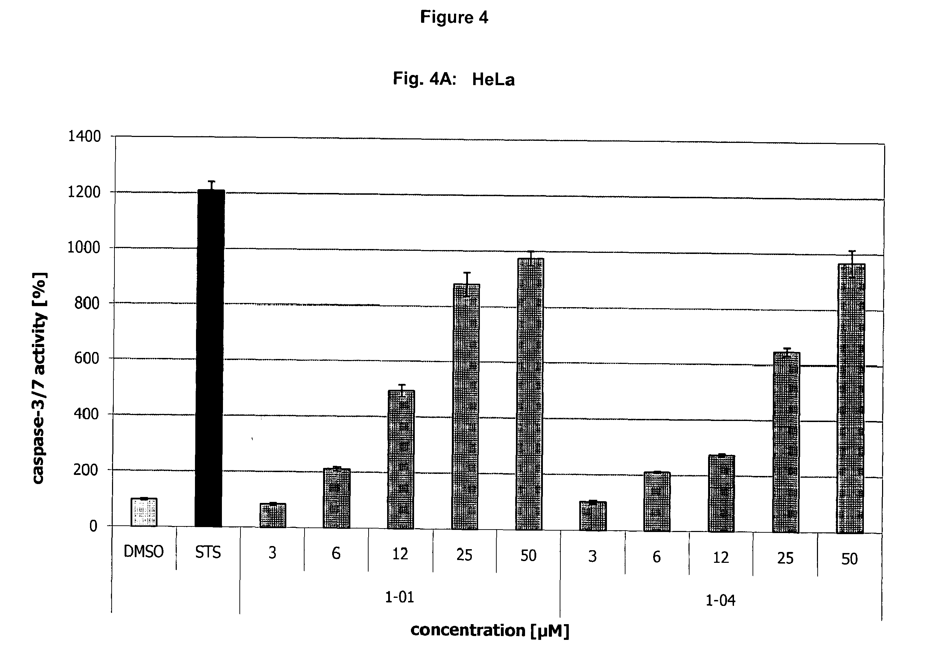

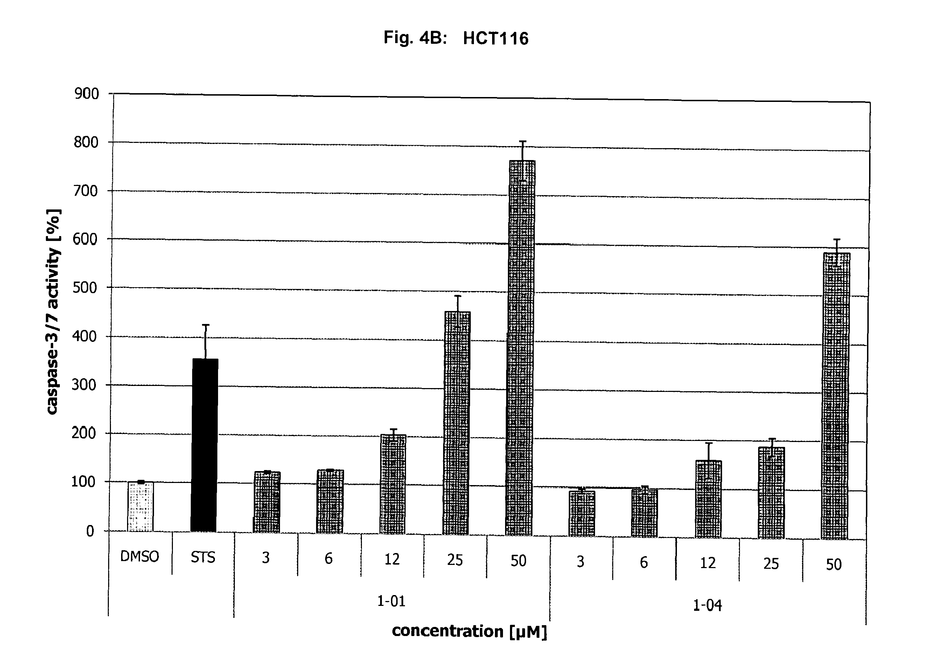

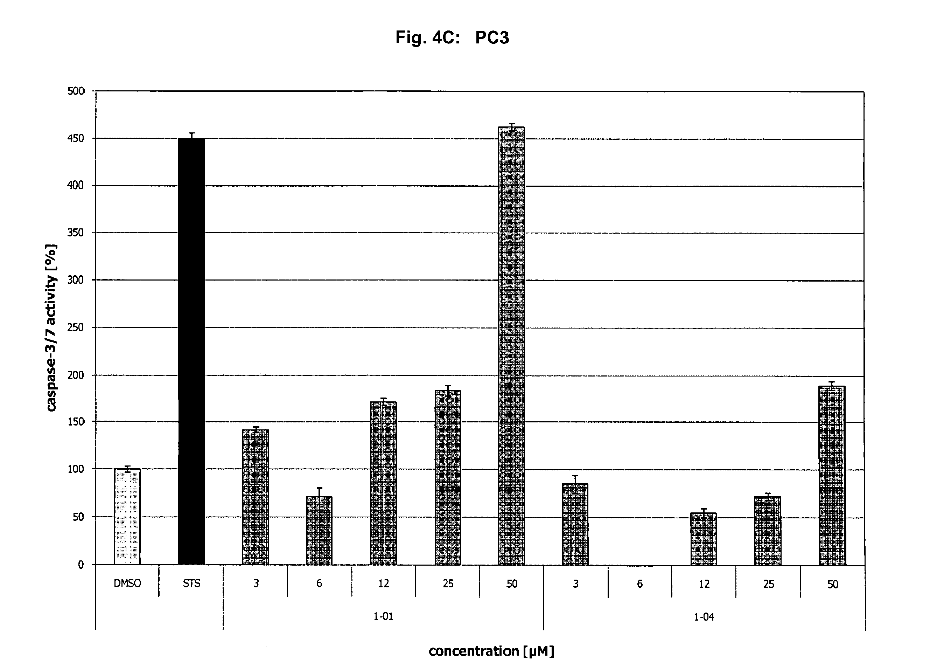

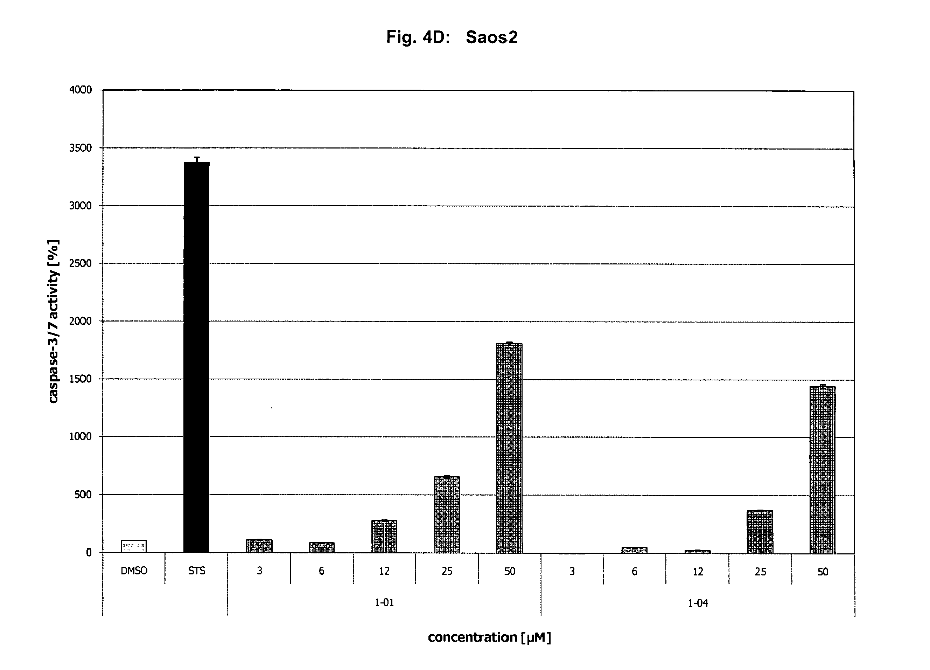

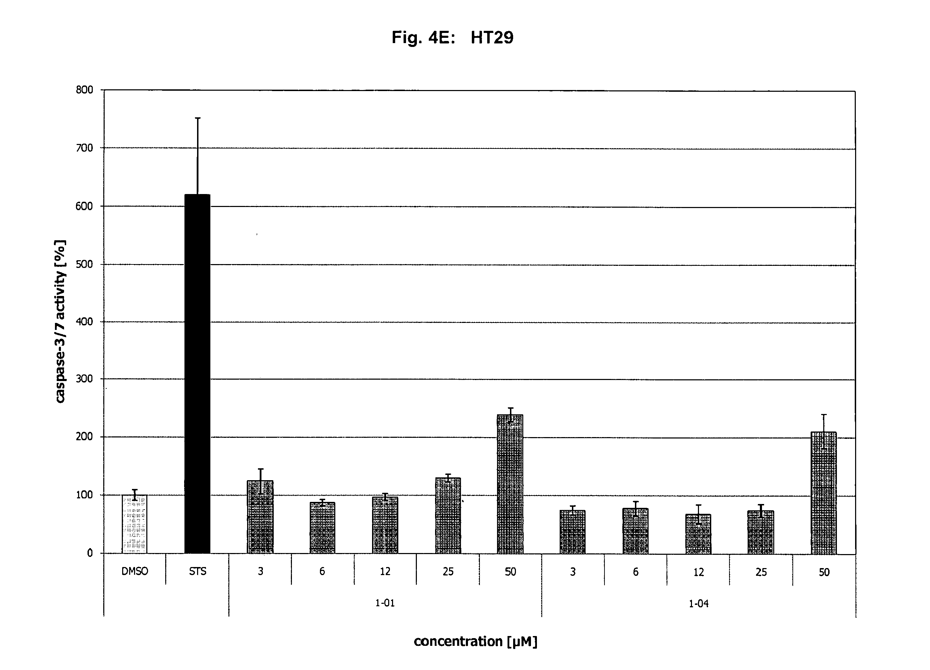

For HeLa, HCT116 and Saos2 cells a concentration dependent increase in the activation of the enzymes was detected for (-)**-1-01** and (-)-**1-04** after 42 h of treatment (Figure 4). For PC3 and HT29 cell lines we could detect caspase-3/7 activity only at the 50 µM concentration. These findings correlate with our results from the-cell viability assays for these indoloquinolizine. In all cell lines but Saos2 the tested inventive compounds (-)**-1-01** and (-)-**1-04** and especially the (+) enantiomers of **1-01** and **1-04** did not have any impact on the apoptosis, which correlates with the cell viability and phenotype studies.

## Claims

1. Compounds having the general formula (I): 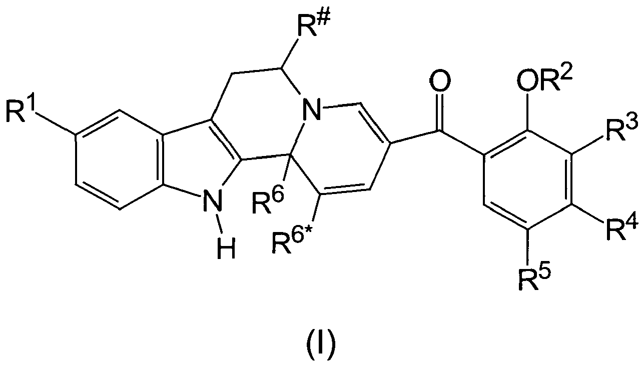 wherein
**R¹** represents -H, -NO₂, -F, -Cl, -Br, -I, -CH₃, -C₂H₅, -C₃H₇, -CH(CH₃)₂, -C₄H₉, -R⁷, -OCH₃, -OC₂H₅, -OC₃H₇, -OCH(CH₃)₂, -OC₄H₉, -OR⁷;
**R², R⁷, R⁸, R⁹-R¹⁵** represent independently of each other -H, -CH₃, -C₂H₅, -CH₂-CH₂F, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -C₃H₇, -CH(CH₃)₂, -C₄H₉, -C₅H₁₁, -C₆H₁₃, -C(CH₃)₃, -CH₂-CH(CH₃)₂, -CH(CH₃)-C₂H₅, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -C₄H₈-CH=CH₂, -C≡CH, -C≡C-CH₃, -CH₂-C≡CH, -C₂H₄-C≡CH;
**R³, R⁴, R⁵, R⁹, R¹⁰, R¹¹, R¹²** represent independently of each other -OR⁸, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cydo-C₃H₅)₂, -NH-CO-N[CH(CH₃)₂]₂, -NH-CO-N[C(CH₃)_{3]2}, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cydo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -C₅H₁₁, -C₆H₁₃, -C(CH₃)₃, -CH₂-CH(CH₃)₂, -CH(CH₃)-C₂H₅, -CH(CH₃)-C₃H₇, -CH₂-CH(CH₃)-C₂H₅, -CH(CH₃)-CH(CH₃)₂. -C(CH₃)₂-C₂H₅, -CH₂-C(CH₃)₃, -CH(C₂H₅)₂, -C₂H₄-CH(CH₃)₂, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂₇-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -C₄H₈-CH=CH₂, -C≡CH, -C≡C-CH₃, -CH₂-C≡CH, -C₂H₄-C≡CH, -P(O)(OH)₂, -P(O)(OCH₃)₂, -P(O)(OC₂H₅)₂, -P(O)(OCH(CH₃)₂)₂, -C(OH)[P(O)(OH)₂]₂;
**R³** and **R⁴** form together a carbocyclic ring represented by 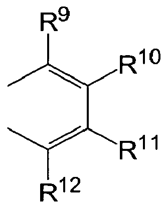
**R⁶** and **R^{6*}** represent independently of each other -H, -COOR¹³, -COOR¹⁴, -R¹³, -R¹⁴, -CN;
**R^{#}** represents -H or -COOR¹⁵;
and enantiomers, stereoisomeric forms, mixtures of enantiomers, diastereomers, mixtures of diastereomers, prodrugs, tautomers, hydrates, solvates and racemates of the above-mentioned compounds and pharmaceutically acceptable salts thereof.

2. Compounds according to claim 1, wherein
**R¹** represents -H, -NO₂, -Cl, -Br, -OH, -R⁷, -OR⁷;
**R³, R⁴** and **R⁵** are hydrogen;
**R⁶** and **R^{6*}** represent independently of each other -H, -COOR¹³, -COOR¹⁴, -R¹³ , -R¹⁴, -CN;
**R^{#}** represents -H;
**R², R⁷, R⁸, R¹³** and **R¹⁴** have the meanings as disclosed in claim 1.

3. Compounds according to claim 1, wherein
**R¹** represents -H, -Cl, -Br, -OH, -CH₃, -C₂H₅, -OCH₃, -OC₂H₅;
**R³, R⁴** and **R⁵** are hydrogen;
**R⁶** and **R^{6*}** represent independently of each other -H, -COOCH₃, -COOC₂H₅, -CH₃, -C₂H₅ or -CN;
**R^{#}** represents -H;
**R²** represents -H, -CH₃ or -C₂H₅.

4. Compounds according to claim 1:
Compound 1-01: 3-(2-Hydroxybenzoyl)-7,12-dihydro-6*H*-indolo[2,3-*a*]quinolizine-1,12b-dicarboxylic acid dimethyl ester,
Compound 1-02: 3-(2-Hydroxybenzoyl)-7,12-dihydro-6*H*-indolo[2,3-*a*]quinolizine-1,12b-dicarboxylic acid diethyl ester,
Compound 1-03: 3-(2-Hydroxybenzoyl)-7,12-dihydro-6*H*-indolo[2,3-*a*]quinolizine-1,12b-dicarboxylic acid diethyl ester,
Compound 1-04: 3-(2-Hydroxy-5-methylbenzoyl)-7,12-dihydro-6*H*-indolo[2,3-*a*]quinolizine-1,12b-dicarboxylic acid diethyl ester,
Compound 1-05: 3-(5-Bromo-2-hydroxybenzoyl)-9-methoxy-7,12-dihydro-6*H-*indolo[2,3-*a*]quinolizine-1,12b-dicarboxylic acid dimethyl ester,
Compound 1-06: 3-(5-Chloro-2-hydroxybenzoyl)-7,12-dihydro-6*H*-indolo[2,3-*a*]quinolizine-1,12b-dicarboxylic acid dimethyl ester,
Compound 1-07: 3-(5-Bromo-2-hydroxybenzoyl)-7,12-dihydro-6*H*-indolo[2,3-*a*]quinolizine-1,12b-dicarboxylic acid dimethyl ester,
Compound 1-08: 3-(2-Hydroxy-5-methylbenzoyl)-7,12-dihydro-6*H*-indolo[2,3-*a*]quinolizine-1,12b-dicarboxylic acid dimethyl ester,
Compound 1-09: 3-(2-Hydroxy-5-iso-propylbenzoyl)-7,12-dihydro-6*H*-indolo[2,3-*a*]quinolizine-1,12b-dicarboxylic acid dimethyl ester,
Compound 1-10: 3-(3,5-Dichloro-2-hydroxybenzoyl)-7,12-dihydro-6*H*-indolo[2,3-*a*]quinolizine-1,12b-dicarboxylic acid dimethyl ester,
Compound 1-11: 3-(3,5-Dibromo-2-hydroxybenzoyl)-7,12-dihydro-6*H*-indolo[2,3-*a*]quinolizine-1,12b-dicarboxylic acid dimethyl ester,
Compound 1-12: 3-(5-Chloro-2-hydroxy-4-methylbenzoyl)-7,12-dihydro-6*H-*indolo[2,3-a]quinolizine-1,12b-dicarboxylic acid dimethyl ester,
Compound 1-13: 3-(2-Hydroxybenzoyl)-9-methoxy-7,12-dihydro-6*H*-indolo[2,3-*a*]quinolizine-1,12b-dicarboxylic acid diethyl ester,
Compound 1-14: 3-(5-Bromo-2-hydroxybenzoyl)-7,12-dihydro-6*H*-indolo[2,3-*a*]quinolizine-1,12b-dicarboxylic acid diethyl ester,
Compound 1-15: 3-(3,5-Dichloro-2-hydroxybenzoyl)-7,12-dihydro-6*H*-indolo[2,3-*a*]quinolizine-1,12b-dicarboxylic acid diethyl ester,
Compound 1-16: 3-(2-Hydroxy-5-methylbenzoyl)-9-methoxy-7,12-dihydro-6*H-*indolo[2,3-*a*]quinolizine-1,12b-dicarboxylic acid diethyl ester,
Compound 1-17: 3-(2-Hydroxy-5-iso-propylbenzoyl)-9-methoxy-7,12-dihydro-6*H-*indolo[2,3-*a*]quinolizine-1,12b-dicarboxylic acid diethyl ester,
Compound 1-18: 3-(5-Chloro-2-hydroxy-4-methylbenzoyl)-7,12-dihydro-6*H-*indolo[2,3-*a*]quinolizine-1,12b-dicarboxylic acid diethyl ester,
Compound 1-19: 3-(5-Chloro-2-hydroxybenzoyl)-7,12-dihydro-6*H*-indolo[2,3-*a*]quinolizine-1,12b-dicarboxylic acid diethyl ester,
Compound 1-20: 3-(5-Chloro-2-hydroxybenzoyl)-9-methoxy-7,12-dihydro-6*H-*indolo[2,3-*a*]quinolizine-1,12b-dicarboxylic acid diethyl ester,
Compound 1-21: 3-(1-Hydroxynaphthalene-2-carbonyl)-7,12-dihydro-6*H-*indolo[2,3-*a*]quinolizine-1,12b-dicarboxylic acid dimethyl ester,
Compound 1-22: 3-(5-Bromo-2-hydroxybenzoyl)-9-methoxy-7,12-dihydro-6*H-*indolo[2,3-*a*]quinolizine-1,12b-dicarboxylic acid diethyl ester,
Compound 1-23: 3-(4-Benzyloxy-2-hydroxy-5-methylbenzoyl)-7,12-dihydro-6*H-*indolo[2,3-*a*]quinolizine-1,12b-dicarboxylic acid dimethyl ester,
Compound 1-24: 3-(2-Methoxybenzoyl)-7,12-dihydro-6H-indolo[2,3-*a*]quinolizine-1,12b-dicarboxylic acid dimethyl ester.
Compound 1-25: Dimethyl-3-(2-hydroxybenzoyl)-9-hydroxy-6,7,12,12b-tetra-hydroindolo[2,3-*a*]quinolizine-1,12b-dicarboxylate
Compound 1-26: Dimethyl-3-(2-hydroxybenzoyl)-9-methyl-6,7,12,12b-tetra-hydro-indolo[2,3-*a*]quinolizine-1,12b-dicarboxylate
Compound 1-27: Dimethyl-9-bromo-3-(2-hydroxybenzoyl)-6,7,12,12b-tetra-hydro-indolo[2,3-*a*]quinolizine-1,12b-dicarboxylate
Compound 1-28: (6*S*)-trimethyl 3-(2-hydroxybenzoyl)-6,7,12,12b-tetrahydro-indolo[2,3-a]quinolizine-1,6,12b-tricarboxylate
Compound 1-29: 3-(2-Hydroxybenzoyl)-7,12-dihydro-6*H*-indolo[2,3-a]quinolizine-1-carboxylic acid methyl ester,
Compound 1-30: 3-(5-Chloro-2-hydroxybenzoyl)-7,12-dihydro-6*H*-indolo[2,3-*a*]quinolizine-1-carboxylic acid methyl ester
Compound 1-31: 3-(2-Hydroxybenzoyl)-7,12-dihydro-6*H*-indolo[2,3-*a*]quinolizine-1-carbonitrile
Compound 1-32: 3-(5-Chloro-2-hydroxybenzoyl)-7,12-dihydro-6*H*-indolo[2,3-*a*]quinolizine-1-carbonitrile
Compound 1-33: 3-(2-Hydroxybenzoyl)-12b-methyl-7,12-dihydro-6*H*-indolo[2,3-*a*]quinolizine-1-carboxylic acid methyl ester
Compound 1-34: 3-(5-Chloro-2-hydroxybenzoyl)-12b-methyl-7,12-dihydro-6*H-*indolo[2,3-*a*]quinolizine-1-carboxylic acid methyl ester
Compound 1-35: 3-(2-Hydroxybenzoyl)-12b-methyl-7,12-dihydro-6*H*-indolo[2,3-*a*]quinolizine-1-carbonitrile
Compound 1-36: 3-(5-Chloro-2-hydroxybenzoyl)-12b-methyl-7,12-dihydro-6*H-*indolo[2,3-*a*]quinolizine-1-carbonitrile
Compound 1-37: 12b-ethyl 1-methyl 3-(2-hydroxybenzoyl)-6,7,12,12b-tetrahydroindolo[2,3-a]quinolizine-1,12b-dicarboxylate
Compound 1-38: 12b-ethyl 1-methyl 3-(5-chloro-2-hydroxybenzoyl)-6,7,12,12b-tetrahydroindolo[2,3-a]quinolizine-1,12b-dicarboxylate
Compound 1-39: ethyl 1-cyano-3-(2-hydroxybenzoyl)-6,7,12,12b-tetrahydroindolo[2,3-a]quinolizine-12b-carboxylate
Compound 1-40: ethyl 3-(5-chloro-2-hydroxybenzoyl)-1-cyano-6,7,12,12b-tetrahydroindolo[2,3-a]quinolizine-12b-carboxylate
Compound 1-41: 3-(2-Hydroxybenzoyl)-9-methoxy-7,12-dihydro-6*H*-indolo[2,3-*a*]quinolizine-1-carboxylic acid methyl ester
Compound 1-42: methyl 3-(2-hydroxybenzoyl)-12b-methyl-6,7,12,12b-tetrahydroindolo[2,3-a]quinolizine-1-carboxylate
Compound 1-43: 3-(2-Hydroxybenzoyl)-9-methoxy-7,12-dihydro-6*H*-indolo[2,3-a]quinolizine-1-carbonitrile
Compound 1-44: 3-(5-Chloro-2-hydroxybenzoyl)-9-methoxy-7,12-dihydro-6*H-*indolo[2,3-*a*]quinolizine-1-carbonitrile
Compound 1-45: 3-(2-Hydroxybenzoyl)-9-methoxy-12b-methyl-7,12-dihydro-6*H-*indolo[2,3-*a*]quinolizine-1-carboxylic acid methyl ester
Compound 1-46: 3-(5-Chloro-2-hydroxybenzoyl)-9-methoxy-12b-methyl-7,12-dihydro-6*H*-indolo[2,3-*a*]quinolizine-1-carboxylic acid methyl ester Compound 1-47: 3-(2-Hydroxybenzoyl)-9-methoxy-12b-methyl-7,12-dihydro-6*H-*indolo[2,3-*a*]quinolizine-1-carbonitrile
Compound 1-48: 3-(5-Chloro-2-hydroxybenzoyl)-9-methoxy-12b-methyl-7,12-dihydro-6*H*-indolo[2,3-a]quinolizine-1-carbonitrile
Compound 1-49: 3-(2-Hydroxybenzoyl)-7,12-dihydro-6*H*-indolo[2,3-a]quinolizine,
Compound 1-50: 3-(2-Hydroxybenzoyl)-12b-methyl-7,12-dihydro-6*H*-indolo[2,3-a]quinolizine
Compound 1-51: 3-(2-Hydroxybenzoyl)-7,12-dihydro-6*H*-indolo[2,3-*a*]quinolizine-12b-carboxylic acid ethyl ester,
Compound 1-52: 3-(2-Hydroxybenzoyl)-9-methoxy-7,12-dihydro-6*H*-indolo[2,3-a]quinolizine
Compound 1-53: 3-(2-Hydroxybenzoyl)-9-methoxy-12b-methyl-7,12-dihydro-6*H-*indolo[2,3-*a*]quinolizine

5. Compounds according to any one of claims 1 - 4 for use as pharmaceutically active agent in medicine.

6. Use of a compound according to any one of claims 1 - 5 for manufacturing a pharmaceutical composition for the induction of apoptosis and for the induction of cell cycle stop.

7. Use of a compound according to any one of claims 1 - 5 for manufacturing a pharmaceutical composition for treatment or prophylaxis of proliferative diseases, tumors or cancer.

8. Use according to claim 7, wherein the proliferative disease, tumor or cancer is selected from the group comprising: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

9. Pharmaceutical composition comprising at least one compound according to claim 1 as an active ingredient, together with at least one pharmaceutically acceptable carrier, excipient and/or diluent.

10. Method for synthesizing the compounds according to claim 1, comprising the following steps (GP I):
Reacting the aldehyd compound A (step 1) 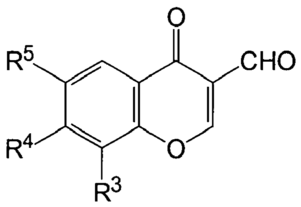
with the compound B 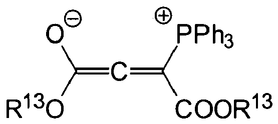
which is in situ generated from triphenylphosphine and acetylenedicarboxylate in order to obtain compound C 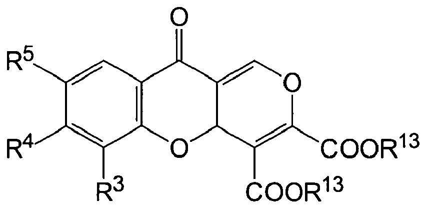
which is reacted with the amine compound D (step2) 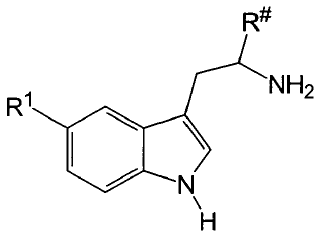
to the final compound of general formula (I), wherein R² is hydrogen (step 3) 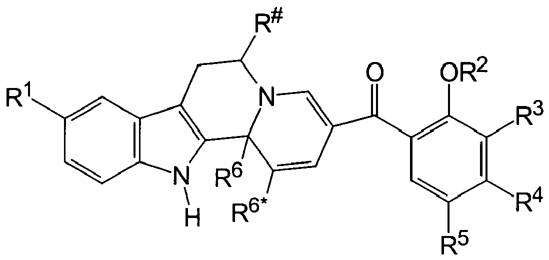
and optionally alkylating the free hydroxy group -OR² in order to obtain the final compounds of general formula (I) wherein R² is different from hydrogen (step 4).

11. The method according to claim 10, wherein in step 3 of the method a chiral organic acid or a chiral phosphoric acid or a chiral sulphonic acid is used.

12. Method for synthesizing the compounds according to claim 1, comprising the following steps (GP IA):
Reacting the compound F (step 1) 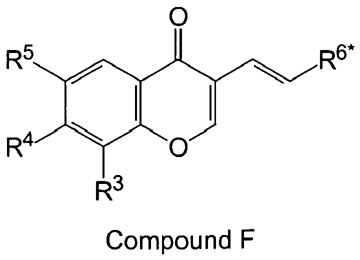
with the amine compound G of the following general formula 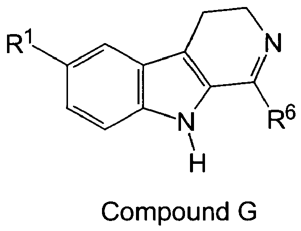
to the final compound of general formula (IA), wherein R² is hydrogen 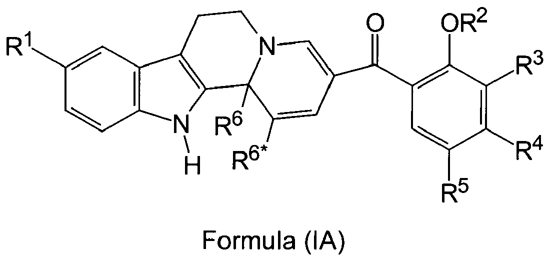
and optionally alkylating the free hydroxy group -OR² in order to obtain the final compounds of general formula (I) wherein R² is different from hydrogen (step 2).
